Europäisches Patentamt

European Patent Office

Office européen des brevets

(11) Numéro de publication : **0 332 478 B1**

(12) **FASCICULE DE BREVET EUROPEEN**

(45) Date de publication du fascicule du brevet :
**25.03.92 Bulletin 92/13**

(51) Int. Cl.⁵ : **A61K 9/50,** A61K 7/00,
A61K 35/78

(21) Numéro de dépôt : **89400177.5**

(22) Date de dépôt : **23.01.89**

(54) **Liposomes contenant des extraits de Scutellaria.**

Le dossier contient des informations
techniques présentées postérieurement au
dépôt de la demande et ne figurant pas dans le
présent fascicule.

(30) Priorité : **09.03.88 FR 8803066**

(43) Date de publication de la demande :
**13.09.89 Bulletin 89/37**

(45) Mention de la délivrance du brevet :
**25.03.92 Bulletin 92/13**

(84) Etats contractants désignés :
**AT BE CH DE ES FR GB GR IT LI LU NL SE**

(56) Documents cités :
**EP-A- 0 224 837**
**PATENT ABSTRACTS OF JAPAN, vol. 10, no.
208 (C-361)[2264], 22 juillet 1986, page 208 C
361; & JP-A-61 50 918 (ICHIMARU FUARU-
KOSU K.K.) 13-03-1986**

(56) Documents cités :
**PATENT ABSTRACTS OF JAPAN, vol. 4, no.
186 (C-36)[668], 20 décembre 1980, page 141 C
36; & JP-A-55 127 309 (HADASHIYOUHIN
KAGAKU KAIHOU KENKYUSHO K.K.) 02-
10-1980
CHEMICAL ABSTRACTS, vol. 93, no. 5, 4 août
1980, page 36, résumé no. 36862s, Columbus,
Ohio, US; O.L. PODHAJCER et al.:" "Effect of
liposome-encapsulated quercetin on DNA
synthesis, lactate production, and cyclic adenosine 3':5'-monophosphate level in Ehrlich
ascites tumor cells", & CANCER RES. 1980,
40(4), 1344-50**

(73) Titulaire : **LVMH RECHERCHE
48-50, rue de Seine, B.P. 79
F-92703 Colombes Cédex (FR)**

(72) Inventeur : **Meybeck, Alain
Les Poissons - Apt. 242 20ter, rue de Bezons
F-92400 Courbevoie (FR)**

(74) Mandataire : **Portal, Gérard et al
Cabinet Beau de Loménie 55, rue
d'Amsterdam
F-75008 Paris (FR)**

Jouve, 18, rue Saint-Denis, 75001 PARIS

## Description

La présente invention concerne essentiellement une composition à base de phases lamellaires lipidiques hydratées ou de liposomes contenant un extrait de Scutellaria, ou au moins l'un de ses constituants, et une composition cosmétique ou pharmaceutique, notamment dermatologique, à activité anti-allergique, anti-inflammatoire, anti-vieillissement, l'incorporant.

Les variétés végétales de Scutellaria de la famille des Labiatae incluent sans limitation Scutellaria Baicalensis, Scutellaria Viscidula ou Scutellaria Galericulata.

Les extraits les plus connus de Scutellaria sont des extraits de racines de Scutellaria Baicalensis Georgi (Scutellaria Radix) dénommés également "OGON" ou même "OUGON".

Des extraits d'Ogon tels que ci-dessus définis sont largement décrits dans la littérature pour une utilisation cosmétique, pharmaceutique et horticole.

On décrit dans la demande de brevet japonais JP-57-209895 publiée sous le n° JP-A-59-101412 une composition cosmétique de protection des cheveux contenant un extrait d'Ogon (racine de Scutellaria Baicalensis).

De même, la demande de brevet japonais JP-57-183419 publiée sous le n° JP-A-59-73509 décrit une composition cosmétique contenant de l'Ogon en poudre à raison de 0,005-2 % en poids, ou un extrait de celui-ci comme un composant essentiel, cette composition présentant un effet excellent d'amélioration des peaux sèches, des taches, des rides, etc. Dans cette demande, on précise que l'Ogon est constitué de racines séchées de Scutellaria Baicalensis Georgi ou de végétaux apparentés.

L'utilisation d'extrait de Scutellaria Radix pour préparer une composition cosmétique évitant la formation de taches sur la peau est encore décrite dans la demande de brevet japonais JP-59-241641 publiée sous le n° JP-A-61-122209, en mélange avec d'autres composants actifs.

L'emploi de Scutellaria comme l'un des composant d'un extrait multicomposant pour la formation d'un réactivateur de la peau est décrit dans la demande de brevet japonais JP-54-172382 publiée sous le n° JP-A-56-92821.

Il a également été décrit dans la demande de brevet japonais JP-54-34771 publiée sous le n° JP-A-55-127309 une composition cosmétique comprenant un extrait de Scutellaria Baicalensis Georgi pour la prévention de la dureté, des brûlures du soleil, et de l'inflammation de la peau. La formulation cosmétique peut être utilisable sous forme de lotion, de crème, d'émulsion, de crème de nettoyage et de savon.

Des compositions pharmaceutiques contenant de l'Ogon sont décrites respectivement dans JP-A-62-26229 (Scutellaria Radix, agent de promotion de différentiation des cellules nerveuses) ; JP-A-61-167623 (Ougon, racine de Scutellaria Baicalensis, agent d'inhibition de coagulation des plaquettes) ; JP-A-61-161219 (Scutellaria Baicalensis Georgi ou Scutellaria Viscidula Buxge, traitement des dermatites atopiques) ; JP-A-61-109733 (Ougon, racine de Scutellaria Baicalensis, agent carcinostatique) ; JP-A-61-263923 (inducteur d'interleukine-2 de faible toxicité contenant, dans un mélange d'extrait d'herbe, un extrait d'Ogon) ; JP-A-58-121218 (composition pour le contrôle de caries dentaires comprenant, entre autres, un extrait de Scutellaria Baicalensis ; GB-A-1096708 (médicament antinarcotique contenant, entre autres, des racines de Scutellaria Baicalensis Georgi (5 %)) ; JP-A-62-033125 (médicament améliorant l'effet anticancéreux de Tégafur contenant, entre autres, des racines de Scutellaria en poudre).

Des extraits d'Ogon ont également été utilisés dans l'agriculture pour la production d'un agent d'activation de la croissance des plantes JP-A-61-115009 ; comme fongicide JP-A-56-022709 ; ou comme agent de contrôle des maladies des plantes, JP-A-62-129209.

Egalement, certains agents actifs ont été extraits de l'Ogon tels que la baïcaline comme constituant pour le traitement des maladies allergiques (voir JP-A-61-50921), la baïcaline ou la baïcaléine encore comme agent anti-allergique (JP-A-61-50918), ainsi que Planta Medica, J. Medici. Plant Research 1981, vol 43, pages 194-201. Comme composant déodorant, il a également été utilisé la baïcaléine ou bäcaline (JP-A-61-268259) ; des dérivés de baïcaléine sous forme de sel et de demi-ester comme agent anti-inflammatoire ou anti-asthmatique sont décrits dans JP-A-70-25716 = US-3549662. L'emploi de wogonine et de baïcaline est décrit dans JP-A-48-68717 pour le traitement de l'artériosclérose, l'apoplexie, et l'hyperchlolestérolémie.

L'emploi de baïcaline, de wogonine, etc., est décrit pour la préparation de pigment anthocyanique dans JP-A-55-13711.

Enfin, l'emploi de Scutellaria dans les compositions cosmétiques pour retenir l'eau de la peau est décrit dans JP-A-60-258104.

On peut ainsi constater que l'emploi de Scutellaria et en particulier d'extrait d'Ogon, ou de divers constituants de celui-ci est largement décrit dans le domaine cosmétique ou pharmaceutique.

Par ailleurs, on connaît déjà l'emploi de phases lamellaires lipidiques hydratées ou de liposomes dans des compositions pharmaceutiques ou des compositions cosmétiques dans lesquelles on incorpore divers principes actifs (FR-A-2 540 381).

Il a maintenant été découvert, de manière tout à fait surprenante et inattendue, que l'incorporation d'extrait précité de Scutellaria en particulier d'extrait d'Ogon, ou de substance active ayant pu être isolée d'un tel extrait d'Ogon ou obtenue par synthèse chimique, choisie parmi : 2′,5,7-trihydroxy-8-méthoxy-flavone (ou 2′-hydroxy-wogonine), 2′,5-dihydroxy-6,6′,7,8-tétraméthoxy-flavone (ou skullcap flavone II ou néobaïcaléine), 2′,5,5′,7-tétrahydroxy-6′,8-diméthoxy-flavone, 5-hydroxy-8-méthoxy-flavone-7-O-D-glucuronide (ou wogonin-7-O-D-glucuronide ou oroxindine), 5-hydroxy-7,8-diméthoxy-flavone (ou 7-O-méthyl-wogonine), 5,7-dihydroxy-6-méthoxy-flavone (ou oroxyline A ou 6-O-méthyl-baïcaléine), 4′,5,7-trihydroxy-8-méthoxy-flavone (ou 4′hydroxy-wogonine), 2′,5,6′-trihydroxy-7,8-diméthoxy-flavone, 5,7,8-trihydroxy-flavone (ou norwogonine), 5,6,7-trihydroxy-flavone (ou baïcaléine), 5,8-dihydroxy-6,7-diméthoxy-flavone, 2′,3,5,6′,7-pentahydroxy-flavone, 4′,5,6-trihydroxy-flavone-7-O-D-glucuronide (ou 4′-hydroxy-baïcaline), acide 5,6-dihydroxy-flavone-7-O-D-glucuronique méthyl ester (ou baïcaline méthyl ester), 2′,5,7-trihydroxy-flavone (2′-hydroxy chrysine), 5,7-dihydroxy-8-méthoxy-flavone (ou wogonine), 2′,5,7-trihydroxy-6′,8-diméthoxy-flavone (ou 2′-hydroxy-6′-méthoxy-wogonine), 4′,5,6,7-tétrahydroxy-flavone (ou 4′-hydroxy-baïcaléine), 5,6-dihydroxy-flavone-7-O-D-glucoside (baïcaléine-7-O-D-glucoside), 5-hydroxy-4′,6,7-tri-méthoxy-flavone (ou salvigénine), 5-hydroxy-6-méthoxy-flavone-7-O-D-glucuronide (ou oroxyline A-7-O-D-glucuronide), 5,6-dihydroxy-flavone-7-O-D-glucuronide (ou baïcaline), acide 5-hydroxy-6-méthoxy-flavone-7-O-D-glucuronique méthyl ester (ou oroxindine méthyl ester), 5,7-dihydroxy-flavone (ou chrysine), et de préférence parmi : wogonine, 2′-hydroxy-wogonine, baïcaléine, néobaïcaléine, oroxindine, baïcaline , au moins en partie dans une phase lamellaire lipidique hydratée ou dans des liposomes, provoquait une activité exacerbée de cet extrait ou de ces substances. Une amélioration radicale d'activité a été observée en ce qui concerne l'activité anti-inflammatoire, l'activité anti-allergique et l'activité anti-vieillissement.

On peut ainsi en déduire, en quelque sorte, un effet de synergie pour des incorporations d'extrait de Scutellaria en particulier d'extrait d'Ogon ou des substances actives isolées de tels extraits comme les substances précitées, dans des phases lamellaires lipidiques hydratées ou dans des liposomes.

Ainsi, la présente invention a pour but de résoudre le nouveau problème technique consistant en la fourniture d'une nouvelle formulation d'extrait de Scutellaria en particulier d'extrait d'Ogon, ou de toute substance active isolée à partir d'un tel extrait ou reconstituée par synthèse chimique, permettant de potentialiser leur efficacité pour permettre leur utilisation dans les compositions cosmétiques ou pharmaceutiques, notamment dermatologiques, à activité anti-inflammatoire ou anti-allergique, ou anti-vieillissement.

La présente invention résout, pour la première fois, ce nouveau problème technique de manière satisfaisante.

Ainsi, selon un premier aspect, la présente invention fournit une composition à base de phases lamellaires lipidiques hydratées ou de liposomes, caractérisée en ce que lesdites phases lamellaires lipidiques hydratées ou lesdits liposomes contiennent au moins en partie un extrait de Scutellaria, ou au moins une substance active isolée d'un tel extrait ou obtenue par synthèse chimique, choisie parmi : 2′,5,7-trihydroxy-8-méthoxy-flavone (ou 2′-hydroxy-wogonine), 2′,5-dihydroxy-6,6′,7,8-tétraméthoxy-flavone (ou skullcap flavone II ou néobaïcaléine), 2′,5,5′,7-tétrahydroxy-6′,8-diméthoxy-flavone, 5-hydroxy-8-méthoxy-flavone-7-O-D-glucuronide (ou wogonin-7-O-D-glucuronide ou oroxindine), 5-hydroxy-7,8-diméthoxy-flavone (ou 7-O-méthyl-wogonine),5,7-dihydroxy-6-méthoxy-flavone (ou oroxyline A ou 6-O-méthyl-baïcaléine), 4′,5,7-trihydroxy-8-méthoxy-flavone (ou 4′-hydroxy-wogonine), 2′,5,6′-trihydroxy-7,8-diméthoxy-flavone, 5,7,8-trihydroxy-fllavone (ou norwogonine), 5,6,7-trihydroxy-flavone (ou baïcaléine), 5,8-dihydroxy-6,7-diméthoxy-flavone, 2′,3,5,6′,7-pentahydroxy-flavone, 4′,5,6-trihydroxy-flavone-7-O-D-glucuronide (ou 4′-hydroxy baïcaline ), acide 5,6-dihydroxy-flavone-7-O-D-glucuronique méthyl ester (ou baïcaline méthyl ester), 2′,5,7-trihydroxy-flavone (2′-hydroxy chrysine), 5,7-dihydroxy-8-méthoxy-flavone (ou wogonine), 2′,5,7-trihydroxy-6′,8-diméthoxy-flavone (ou 2-hydroxy-6′-méthoxy-wogonine), 4′,5,6,7-tétrahydroxy-flavone (ou 4′-hydroxy-baïcaléine), 5,6-dihydroxy-flavone-7-O-D-glucoside (baïcaléine-7-O-D-glucoside), 5-hydroxy-4′,6,7-triméthoxy-flavone (ou salvigénine), 5-hydroxy-6-méthoxy-flavone-7-O-D-glucuronide (ou oroxyline A-7-O-D-glucuronide), 5,6-dihydroxy-flavone-7-O-D-glucuronide (ou baïcaline), acide 5-hydroxy-6-méthoxy-flavone-7-O-D-glucuronique méthyl ester (ou oroxindine méthyl ester), 5,7-dihydroxy-flavone (ou chrysine).

Pour une description précise de ces substances isolées, on peut se reporter à la description de l'art antérieur précédente, notamment Planta Medica, Journal of Medicinal Plant Research (1981), vol. 43, pages 194-201, également au document Chem. Pharm. Bull., (1984), vol. 32, pages 5051-5054 ou encore Chem. Pharm. Bull. (1988), vol. 36 n°2, pages 654-661.

Selon un mode avantageux de réalisation de cette composition, ladite substance active est choisie parmi le groupe constitué par la wogonine, la 2′-hydroxy-wogonine, la baïcaléine, la néo-baïcaléine, l'oroxindine et la baïcaline.

Selon une caractéristique particulière de cette composition, celle-ci contient un extrait de Scutellaria obtenu par une extraction par solvant, de préférence choisi parmi le groupe consistant d'un solvant polaire, en particulier une solution alcoolique ou hydroalcoolique ou éthérée ; d'un solvant organique apolaire comme le n-hexane, le benzène, ou une combinaison des deux.

Selon une variante de réalisation, on effectue en premier lieu une extraction avec un solvant organique polaire, suivie d'une extraction avec un solvant organique apolaire, pour récolter la fraction insoluble dans le solvant apolaire, comme décrit dans Planta Medica, Journal of Medicinal Plant Research, 1981, vol 43, p 194-201.

Selon une variante de réalisation de cette composition, celle-ci est caractérisée en ce que l'extrait précité de Scutellaria seul, ou en mélange avec d'autres substances actives compatibles, est introduit dans la phase lipidique des phases lamellaires lipidiques hydratées ou des liposomes.

Selon une autre variante de réalisation de cette composition, celle-ci est caractérisée en ce que l'extrait précité de Scutellaria seul, ou en mélange avec d'autres substances actives compatibles, est introduit dans la phase aqueuse des phases lamellaires lipidiques hydratées ou des liposomes.

Selon un mode de réalisation particulier, l'extrait de Scutellaria est un extrait choisi parmi le groupe consistant de Scutellaria Baicalensis, de Scutellaria Viscidula , ou de Scutellaria Galericulata. Selon un mode avantageux de réalisation, l'extrait de Scutellaria précité est un extrait de racines de Scutellaria Baicalensis Georgi encore nommé extrait d'Ogon.

Selon un deuxième aspect, la présente invention concerne aussi une composition cosmétique ou pharmaceutique, notamment dermatologique, à activité anti-inflammatoire, anti-allergique ou anti-vieillissement, caractérisée en ce qu'elle comprend une composition à base de phases lamellaires lipidiques hydratées ou de liposomes, telle que précédemment définie.

De préférence, la proportion en poids de l'extrait sec de Scutellaria ou de toute substance active obtenue à partir d'un tel extrait ou par synthèse chimique est comprise entre 0,0001 et 2 % relativement au poids total de la composition ; encore de préférence est comprise entre 0,001 et 0,4 % relativement au poids total de la composition.

On peut également utiliser de manière plus pratique, un extrait brut d'Ogon disponible dans le commerce, notamment en solution hydroalcoolique à 50 %, qui peut alors être utilisé à raison de 0,005 à 50 %, encore mieux entre 0,05 et 20 % en poids par rapport au poids total de la composition.

De même, cette composition pharmaceutique, notamment dermatologique, ou cosmétique peut, selon une première variante, être caractérisée en ce que l'extrait précité de Scutellaria, ou toute substance active extraite de celui-ci, seule ou en mélange avec d'autres substances actives compatibles, est introduit dans la phase lipidique des phases lamellaires lipidiques hydratées ou des liposomes, tandis que, selon une autre variante, cette introduction peut être réalisée dans la phase aqueuse des phases lamellaires lipidiques hydratées ou des liposomes.

Dans la présente description et les revendications, le terme "lipidique" dans l'expression "phase lamellaire lipidique" couvre toutes les substances comprenant une chaîne carbonée dite grasse, généralement supérieure à 5 atomes de carbone.

Selon l'invention, on utilise des lipides amphiphiles, c'est-à-dire constitués de molécules possédant un groupe hydrophile indifféremment ionique ou non ionique et un groupe lipophile, ces lipides amphiphiles étant susceptibles de former des phases lamellaires lipidiques en présence d'une phase aqueuse. En particulier, citons parmi ces lipides : les phospholipides, les phosphoaminolipides, les glycolipides, les alcools gras polyoxyéthylénés, les esters de polyols éventuellement polyoxyéthylénés. De telles substances sont par exemple constituées par une lécithine d'oeuf ou de soja, une phosphatidylsérine, une sphyngomyéline, un cérébroside ou un stéarate de polyglycérol oxyéthyléné.

D'autres buts, caractéristiques et avantages de l'invention apparaîtront clairement à la lumière de la description explicative qui va suivre faite en référence à plusieurs exemples de l'invention. Dans les exemples, les pourcentages sont donnés en poids sauf indication contraire.

Exemple 1 de l'invention

A - Préparation d'une composition sous forme de suspension de liposomes avantageusement homogénéisée

On utilise comme extrait d'Ogon un extrait d'Ogon obtenu à partir de racine de Scutellaria Baicalensis Georgi commercialisé par la société japonaise Ichimaru Pharcos Co. Limited, constitué ici du lot n°IT134, appelé "Woogon extract-E", et qui se présente sous la forme d'une solution hydroalcoolique à 50 % volume/volume dans l'éthanol, ayant une densité de 0,931, une teneur en résidu après évaporation de 1,38 % en poids/volume, une teneur en baïcaline de 0,16 % en poids/volume et une très faible teneur de baïcaléine.

Cet extrait d'Ogon (encore dénommé dans la littérature Ougon ou Woogon) peut être évaporé à sec, ou de manière plus pratique, utilisé tel quel pour préparer une composition sous forme de suspension de liposomes, de la manière suivante :

```
composition
- extrait d'Ogon
  (Woogon  extract  n°IT134,  solution  hydroalcoolique  à  50 %
  d'éthanol)................................... 0,5 g
- eau bidistillée.............................. 47,5 g
- lécithine de soja........................... 2,0 g
```

Cette composition est préparée de la manière suivante :

on ajoute tout d'abord l'extrait d'Ogon dans l'eau bidistillée, sous agitation, puis on disperse la lécithine de soja dans cette solution aqueuse.

Une homogénéisation de cette solution est réalisée en continuant cette agitation pendant environ 2 h.

On réalise de préférence une homogénéisation par ultrasons en effectuant une sonication pendant 10 min à 100 W, ce qui permet d'obtenir une dimension de liposomes moyenne de l'ordre de 106,7 nm ± 0,5 nm.

Au lieu d'une homogénéisation aux ultrasons, on peut réaliser une homogénéisation sous pression, par exemple selon le procédé décrit dans le document FR-A-2 534 487.

On observera qu'on peut réaliser diverses dilutions en modifiant la quantité d'extrait ajoutée au départ ou en augmentant le volume de la solution de dispersion, ce qui constitue un procédé aisé de préparation de diverses concentrations en extrait.

En l'absence d'une dilution, on obtient, après cette étape A, 50 g de suspension homogénéisée correspondant à environ 50 ml.

B - Préparation d'une composition de liposomes homogénéisés sous forme de gel

Cette suspension homogénéisée peut être gélifiée par mélange avec un gel, tel qu'un gel de polymère vinylique, en particulier commercialisé sous la dénomination commerciale Carbopol ® 940.

Ainsi, pour préparer ce gel de façon classique, on peut par exemple disperser 0,5 g de Carbopol ® 940 dans 50 g d'eau en présence d'un conservateur et d'un agent chélatant habituel, puis, après gonflement, on peut, de préférence, neutraliser à pH 7,5 avec de la triéthanolamine.

Ainsi, aux 50 g ou millilitres de suspension homogénéisée obtenus à l'étape A ci-dessus, on ajoute 50 ml dudit gel, pour obtenir un volume total de 100 ml d'environ.

Dans cette composition gélifiée, la concentration en extrait sec d'Ogon est d'environ 0,069 % et la concentration en lécithine est de 2 %.

Cette composition ainsi gélifiée, référencée CI.1, sera utilisée dans les essais d'activité donnés plus loin dans la présente description.

Exemple 2 selon l'invention

Composition de liposomes contenant de l'extrait d'Ogon dans la phase lipidique, éventuellement gélifiée

On prend 1,0 g d'extrait d'Ogon de l'exemple 1 que l'on dissout dans 50 cm³ de chloroforme.

On évapore à sec sous pression réduite dans un évaporateur rotatif à une température de l'ordre de 56°C.

On reprend le résidu déposé sur la paroi du ballon rotatif avec 10 ml de méthanol.

On y ajoute 2 g de lécithine de soja, ainsi que 50 ml de chloroforme.

On évapore la solution complète sous pression réduite dans le même ballon rotatif à une température d'environ 56°C pour obtenir un film qui s'est déposé sur la paroi du ballon rotatif.

On reprend ensuite ce film par 48,0 g d'eau.

On agite pendant 3 h à l'aide d'un agitateur magnétique pour obtenir ainsi une suspension de liposomes contenant l'extrait d'Ogon au moins en partie dans la phase lipidique.

On peut homogénéiser les liposomes par sonication, pendant 10 min à 100 W, dans un bain de glace pour obtenir une suspension de liposomes homogénéisés.

On peut, de la même manière qu'à l'exemple 1, gélifier éventuellement cette solution pour obtenir une composition gélifiée ayant une concentration en extrait sec d'Ogon d'environ 0,138 % en poids/volume et une concentration en lécithine de 2 %.

Exemple 3

Baïcaléine en liposomes

On procède comme à l'exemple 2, sauf qu'on utilise 0,1 g de baïcaléine en lieu et place de l'extrait d'Ogon.

Exemple 4

Compositions de comparaison

4-A On prend 0,5 g de l'extrait d'Ogon de l'exemple 1 que l'on ajoute à 49,5 g d'eau bidistillée et on effectue un mélange sous agitation pendant quelques minutes.

Ensuite, on ajoute 5 g de gel de Carbopol ® 940 à 1 % préparé comme décrit à l'exemple 1B de manière à obtenir une composition gélifiée de comparaison référencée C.P n°1.

4-B On prépare encore une composition de comparaison témoin référencée C.P n°2 en mélangeant 50 g d'eau bidistillée et 50 g de gel de Carbopol ® 940 à 1 % préparé comme décrit à l'exemple 1.

4-C On prépare encore une composition de comparaison liposomale sans substance active de la manière indiquée à l'exemple 1, si ce n'est que l'on ne met pas d'extrait d'Ogon. Cette composition est donc formée de 2 g de lécithine de soja, 47,5 g d'eau bidistillée, puis la suspension qui a été homogénéisée aux ultrasons comme indiqué à l'exemple 1A est ensuite gélifiée comme indiqué à l'exemple 1B pour fournir une composition de comparaison référencée C.P n°3.

Exemple 5

Mise en évidence de l'activité anti-allergique et anti-inflammatoire des compositions conformes à l'invention

On vérifie l'utilisation de la composition de l'exemple 1 à titre de composition pharmaceutique, notamment dermatologique, ou cosmétique en effectuant les expérimentations in vivo suivantes :

1 - Mise en évidence de l'activité anti-allergique

L'activité anti-allergique est testée selon le test au DNCB (chloro-1-dinitro-2,4-benzène) chez le cobaye.

Pour ce faire, on forme 5 lots de 10 cobayes ayant sensiblement le même poids et ne présentant aucun signe d'allergie détectable.

Les 50 cobayes sont sensibilisés au DNCB par une injection de DNCB à 0,2 % en poids par voie intradermique. Une semaine après, ils reçoivent une application topique de la même solution de DNCB à 0,2 %.

Les cobayes sont ensuite laissés au repos pendant 12 j.

Après cette période, on provoque à nouveau la réaction allergique par une nouvelle application sous patch d'une solution de DNCB à 0,02 %.

Pour tester l'activité anti-allergique de la composition selon l'invention de l'exemple n°1 (lot n°1) en comparaison des autres compositions comparatives indiquées ci-après (lot n°2 à lot n°5), les cobayes avaient reçu en application, 1 h avant l'application sous patch, respectivement :

– pour le lot n°1 : 1 ml d'extrait d'Ogon en liposome (CI 1 de l'exemple 1) (Ogon dans liposomes dans gel)

– pour le lot n°2 : 1 ml d'extrait d'Ogon à 0,5 % en gel (CP n°1 de l'exemple 4-A) (Ogon dans gel)

– pour le lot n°3 : 1 ml de gel (C.P n°2 de l'exemple 4-B) (gel)

– pour le lot n°4 : 1 ml de liposomes "vides" en gel (C.P n°3 de l'exemple 4-C)

– pour le lot n°5 : aucune application (lot témoin).

L'intensité de la réaction allergique des animaux est notée de 0 à 5, la note 5 étant attribuée à la réaction observée la plus forte.

Le tableau I ci-dessous indique le nombre d'animaux pour chaque note attribuée.

Tableau I

|  | 0 | 1 | 2 | 3 | 4 | 5 | TOTAL |
|---|---|---|---|---|---|---|---|
| Lot 1 : C.I 1 : ogon dans liposomes dans gel | 1 | 5 | 2 | 1 | 1 | - | 16 |
| Lot 2 : CP 1 : ogon dans gel | - | 4 | 2 | 1 | 3 | - | 23 |
| Lot 3 : CP 2 : gel | 1 | 4 | 1 | 2 | 1 | 1 | 21 |
| Lot 4 : CP 3 : liposomes "vides" dans gel | 2 | 2 | 2 | 2 | 2 | - | 20 |
| Lot 5 : témoin | - | - | - | 2 | 6 | 2 | 40 |

Les résultats répertoriés au tableau I montrent très clairement que les réactions allergiques sont en moyenne beaucoup moins fortes chez les cobayes préalablement traités par la suspension de liposomes contenant de l'extrait d'Ogon (lot n°1), par rapport à celles observées chez les animaux des lots n°2 à n°5 recevant des compositions prises à titre de comparaison (lot n°2 à lot n°4) ou sans aucune application (lot n°5).

De plus, on peut observer aussi que l'extrait d'Ogon apparaît nettement plus actif en liposomes par rapport à la forme libre en gel (lot n°2).

Cet essai permet donc de démontrer de manière incontestable l'activité anti-allergique exacerbée obtenue par l'incorporation d'extrait d'Ogon en phases lamellaires lipidiques hydratées ou en liposomes réalisée selon l'invention.

2 - Mise en évidence de l'activité anti-inflammatoire

On met en évidence l'activité anti-inflammatoire de la composition selon l'invention par un test à l'huile de croton, effectué selon la méthode de TONELLI dans la revue Endocrinology, 1965, volume 77, pages 624-634, réalisé sur la souris albinos, selon le protocole suivant :

Les souris sont réparties en sept lots de 8 souris, chaque lot étant traité par un produit donné :
- le lot N° 1 est traité par de l'Ogon dans des liposomes,
- le lot N° 2 est traité avec des liposomes vides dans le gel,
- le lot N°3 est traité avec de l'Ogon dans le gel,
- le lot N°4 est traité par le gel seul,
- le lot N°5 est traité avec du Dectancyl,
- le lot N°6 est un lot témoin auquel on applique seulement de l'huile de croton,
- le lot N°7 est un autre lot témoin qui ne subit aucune application, donc sans huile de croton.

On applique 0,1 ml du produit à tester sur l'oreille droite 3 h, 2 h et 1 h avant l'application de 0,05 ml d'huile de croton à 0,2 % dans l'acétone.

Pour éviter des phénomènes artéfactuels au niveau de l'absorption des produits, les excipients huileux sont proscrits dans cette étude.

5 h 30 plus tard, les animaux sont sacrifiés.

L'oreille traitée est prélevée et pesée sur une balance de précision (mettler).

La moyenne des poids est effectuée pour chaque lot.

La moyenne des poids témoins sera alors retranchée des résultats obtenus dans les autres lots, pour obtenir les valeurs d'augmentation des poids d'oreilles, par rapport aux animaux normaux.

Le pourcentage de protection des produits testés est alors effectué en ramenant le lot huile de croton à 100 %.

Le tableau II donne les résultats individuels.

Le tableau III présente l'analyse statistique par t de Student.

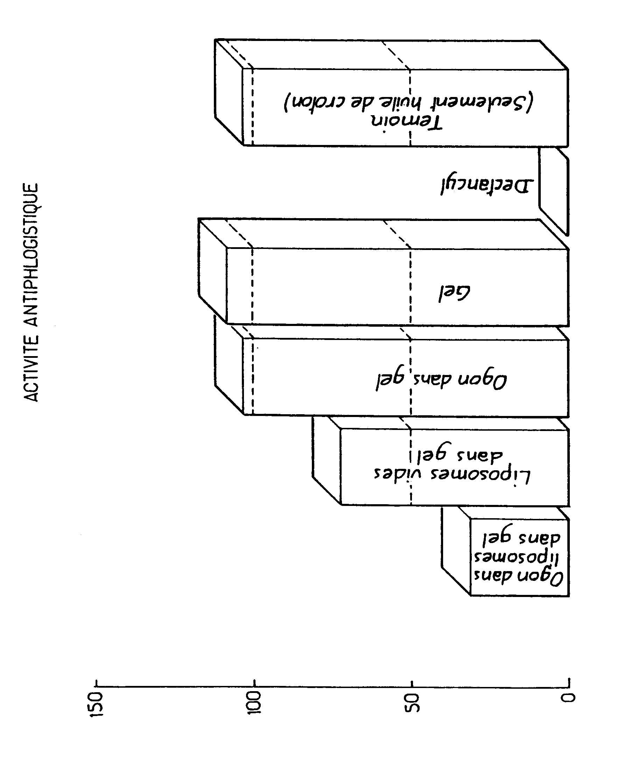

Les résultats du tableau II sont représentés sous la forme d'un histogramme à la figure 1 annexée, la hauteur des barres représente l'importance de la réaction inflammatoire provoquée par l'huile de croton appliquée selon le protocole précédemment indiqué.

On peut observer à partir de ces résultats d'essais que l'oedème provoqué par l'huile de croton (mesuré par pesée de l'oreille) est non significativement diminué par les produits appliqués, sauf l'extrait d'Ogon incorporé dans des liposomes conformément à la présente invention, et le Dectancyl.

L'activité du Dectancyl est significativement supérieure à l'activité des autres produits.

L'extrait d'Ogon en gel, ainsi que le gel seul ou les liposomes "vides" en gel, n'apporte aucune activité antiphlogistique.

On peut, en outre, observer que l'extrait d'Ogon en liposomes selon l'invention apporte une activité antiphlogistique de 69,6 %, ce qui est remarquable et complètement inattendu pour un homme de l'art.

On donne ci-après divers exemples de compositions dermatologiques, dermo-cosmétiques.

Tableau II

| Composition testée | Ogon dans liposomes (ex. 1, CI1) | Liposomes vides dans gel (ex. 3-C) (CP3) | Ogon dans gel (CP1) (ex. 3-A) | Cel seul (ex. 3-B) (CP2) | Dectancyl | Témoin (seulement huile de croton) | Témoin (sans huile de croton) |
|---|---|---|---|---|---|---|---|
| 1 | 138 | 161 | 209 | 278 | 121 | 183 | 134 |
| 2 | 147 | 167 | 194 | 195 | 126 | 192 | 140 |
| 3 | 146 | 255 | 187 | 190 | 123 | 224 | 130 |
| 4 | 160 | 190 | 210 | 240 | 121 | 170 | 122 |
| 5 | 157 | 166 | 208 | 172 | 100 | 188 | 140 |
| 6 | 165 | 172 | 214 | 202 | 108 | 216 | 135 |
| 7 | 167 | 175 | 190 | 185 | 133 | 200 | 133 |
| 8 | 156 | 160 | 194 | 181 | | 238 | 138 |
| MOYENNE | 154,5 | 180,7 | 200,7 | 205,3 | 118,8 | 201,4 | 134 |
| ECART-TYPE | 10,04 | 31,4 | 10,5 | 35,7 | 11,2 | 22,8 | 5,9 |
| MOYENN.PRODUIT MOYENN.TEMOIN | 20,5 | 46,7 | 66,7 | 71,3 | - 15,2 | 67,4 | |
| ECART-TYPE | 15,9 | 36,3 | 16,4 | 41,6 | 17,1 | 28,7 | |
| HC ramené à 100 % | 30,4 | 69,3 | 98,9 | 105,7 | - 22,5 | 100 | |
| PROTECTION | 69,6% | 30,7% | 0 % | - 5,7% | 122,5 | | |

EP 0 332 478 B1

Tableau III

| | OGON DANS LIPOSOMES DANS GEL (Ex.1)(CI1) | LIPOSOMES VIDES DANS GEL (Ex. 3-C)(CP3) | OGON DANS GEL (Ex. 3-A)(CP1) | GEL SEUL (Ex. 3-B)(CP2) | DECTANCYL | TEMOIN (sans huile de croton) |
|---|---|---|---|---|---|---|
| OGON DANS LIPOSOMES DANS GEL (Ex. 1)(CI1) | | >* | >*** | >** | >*** | >*** |
| LIPOSOMES VIDES DANS GEL (Ex. 3-C)(CP3) | | | NS | NS | >*** | NS |
| OGON DANS GEL (Ex. 3-A)(CP1) | | | | NS | >*** | NS |
| GEL SEUL (Ex. 3-B)(CP2) | | | | | >*** | NS |
| DECTANCYL | | | | | | >*** |

t = 2,19    5 % = 2,14    * = 5 %    ** = 1 %    *** = 0,1 %

Exemple 6 : <u>Crème pour peaux sensibles</u>

On réalise un mélange de suspension de liposomes contenant de l'Ogon avec une émulsion de type huile dans eau dans les proportions suivantes :

```
Composition de liposomes préparée
suivant l'exemple 1 à 0,2 % d'ex-
trait sec d'Ogon                              25 g


Excipient émulsionné huile dans eau    qsp   100
```

On réalise une application quotidienne ou biquotidienne par temps froid et sec.

Exemple 7 : <u>Crème pour le soin du contour des yeux</u>

On réalise un mélange de suspension de liposomes contenant de l'Ogon avec une émulsion de type huile dans eau dans les proportions suivantes :

```
Composition de liposomes préparée
suivant l'exemple 2 à 0,15 % d'ex-
trait sec d'Ogon                              30 g


Excipient émulsionné huile dans eau    qsp   100
```

On réalise une application quotidienne sur les paupières inférieure et supérieure.

Exemple 8 : <u>Lait solaire</u>

On réalise un mélange de suspension de liposomes contenant de l'Ogon avec une émulsion de type huile dans eau dans les proportion suivantes :

```
Composition de liposomes préparée
suivant l'exemple 2 à 0,15 % d'ex-
trait sec d'Ogon                              30 g


Excipient émulsionné huile dans
eau chargé en filtres solaires         qsp 100
```

Exemple 9 : <u>Gel de protection pour peaux sujettes aux allergies</u>

```
Composition de liposomes préparée
suivant l'exemple 1 à 0,2 % d'ex-
trait sec d'Ogon                              50 g


Excipient gélifié                      qsp 100
```

Utilisation en application locale quotidienne pour éviter l'apparition de réaction allergique ou en diminuer l'ampleur.

Exemple 10 : Mascara pour paupières sensibles

```
Composition de liposomes préparée
suivant l'exemple 2 à 0,15 % d'ex-
trait sec d'Ogon                           20 g


Emulsion huile dans eau gélifiée
et chargée en pigments              qsp 100
```

Exemple 11 : Composition pour masquer les cernes destinée aux peaux sensibles

```
Composition de liposomes préparée
suivant l'exemple 1 à 0,2 % d'ex-
trait sec d'Ogon                           10 g
Emulsion huile dans eau chargée
en pigments                         qsp 100
```

Exemple 12 : Fond de teint pour peaux sensibles

```
Composition de liposomes préparée
suivant l'exemple 1 à 0,1 % d'ex-
trait sec d'Ogon                           20 g


Emulsion huile dans eau chargée
en pigments                         qsp  100
```

Exemple 13 : Composition anti-allergique et anti-inflammatoire

```
Composition de liposomes préparée
suivant l'exemple 3 à 0,1 % de
baïcaléine                                 20 g


Emulsion huile dans eau            qsp  100
```

Exmeple 14 : Crème de jour pour retarder le vieillissement de la peau

```
Composition de liposome préparée
selon l'exemple 1 à 0,3 d'extrait
sec d'Ogon                                 20 g
Excipient émulsionné huile dans eau  qsp 100
```

Application quotidienne le matin sur les parties du corps exposées à la lumière du jour.


**Revendications**

**Revendications pour les Etats contractants suivants : DE, GB, FR, IT, NL, SE, CH, LI, BE, AT, LU**

1. Composition à base de phases lamellaires lipidiques hydratées ou de liposomes, caractérisée en ce que lesdites phases lamellaires lipidiques hydratées ou lesdits liposomes contiennent au moins en partie un extrait de Scutellaria, ou au moins une substance active isolée d'un tel extrait ou obtenue par synthèse chimique, choisie parmi : 2′,5,7-trihydroxy-8-méthoxy flavone (ou 2′-hydroxy-wogonine), 2′,5-dihydroxy-6,6′,7,8-tétraméthoxy-flavone (ou skullcap flavone II ou néobaïcaléine), 2′,5,5′,7-tétrahydroxy-6′,8-diméthoxy-flavone, 5-hydroxy-8-méthoxy-flavone -7-O-D-glucuronide (ou wogonin-7-O-D-glucuronide ou oroxindine), 5-hydroxy-7,8-diméthoxy-flavone (ou 7-O-méthyl-wogonine), 5,7-dihydroxy-6-méthoxy-flavone (ou oroxyline A ou 6-O-méthyl-baïcaléine), 4′,5,7-trihydroxy-8-méthoxy-flavone (ou 4′-hydroxy-wogonine), 2′,5,6′-trihydroxy-7,8-diméthoxy-flavone, 5,7,8-trihydroxy-flavone (ou norwogonine), 5,6,7-trihydroxy-flavone (ou baïcaléine), 5,8-dihydroxy-6,7-diméthoxy-flavone, 2′,3,5,6′,7-pentahydroxy-flavone, 4′,5,6-trihydroxy-flavone-7-O-D-glucuronide (ou 4′-hydroxy-baïcaline ), acide 5,6-dihydroxy-flavone-7-O-D-glucuronique méthyl ester (ou baïcaline méthyl ester), 2′,5,7-trihydroxy-flavone (2′-hydroxy chrysine), 5,7-dihydroxy-8-méthoxy-flavone (ou wogonine), 2′,5,7-trihydroxy-6′,8-diméthoxy-flavone (ou 2′-hydroxy-6′-méthoxy-wogonine), 4′,5,6,7-tétrahydroxy-flavone (ou 4′-hydroxy-baïcaléine), 5,6-dihydroxy-flavone-7-O-D-glucoside (baïcaléine-7-O-D-glucoside), 5-hydroxy-4′,6,7-triméthoxy-flavone (ou salvigénine), 5-hydroxy-6-méthoxy-flavone-7-O-D-glucuronide (ou oroxyline A-7-O-D-glucuronide), 5,6-dihydroxy-flavone-7-O-D-glucuronide (ou baïcaline), acide 5-hydroxy-6-méthoxy-flavone-7-O-D-glucuronique méthyl ester (ou oroxindine méthyl ester), 5,7-dihydroxy-flavone (ou chrysine).

2. Composition selon la revendication 1, caractérisée en ce que ladite substance active est choisie parmi le groupe constitué par la wogonine, la 2′-hydroxy-wogonine, la baïcaléine, la néobaïcaléine, l'oroxindine et la baïcaline.

3. Composition selon la revendication 1, caractérisée en ce que l'extrait précité est obtenu par une extraction par solvant, de préférence choisi parmi le groupe consistant d'un solvant polaire, notamment une solution alcoolique ou hydroalcoolique, ou une solution éthérée, d'un solvant organique apolaire, comme le n-hexane, le benzène, ou avantageusement une combinaison des deux.

4. Composition selon l'une des revendications 1 à 3, caractérisée en ce que l'extrait précité seul ou la substance active isolée d'un tel extrait ou obtenue par synthèse chimique d'un tel extrait, est utilisé seul ou en mélange avec d'autres substances actives compatibles.

5. Composition selon l'une des revendications 1 à 4, caractérisée en ce que l'extrait précité seul ou ladite substance active isolée d'un tel extrait ou obtenue par synthèse chimique est introduit dans la phase lipidique des phases lamellaires lipidiques hydratées ou des liposomes.

6. Composition selon l'une des revendications 1 à 4, caractérisée en ce que l'extrait précité ou la substance active isolée d'un tel extrait ou obtenue par synthèse chimique précitée est introduit dans la phase aqueuse des phases lamellaires lipidiques hydratées ou des liposomes.

7. Composition selon l'une quelconque des revendications 1 à 6, caractérisée en ce que le Scutellaria est choisi parmis le groupe consistant de Scutellaria Baicalensis, de Scutellaria Viscidula ou de Scutellaria Galericulata.

8. Composition selon l'une des revendications 1 à 7, caractérisée en ce que l'extrait de Scutellaria est un extrait de racines de Scutellaria Baicalensis Georgi encore appelé extrait d'Ogon.

9. Composition cosmétique ou pharmaceutique, notamment dermatologique, à activité anti-inflammatoire, anti-allergique ou anti-vieillissement, caractérisée en ce qu'elle comprend une composition à base de phases lamellaires lipidiques hydratées ou de liposomes contenant au moins en partie un extrait de Scutellaria, ou au moins une substance active isolée d'un tel extrait ou obtenue par synthèse chimique, choisie parmi : 2′,5,7-trihydroxy-8-méthoxy-flavone (ou 2′-hydroxy-wogonine), 2′,5-dihydroxy-6,6′,7,8-tétramèthoxy-flavone (ou skullcap flavone II ou néobaïcaléine), 2′,5,5′,7-tétrahydroxy-6′,8-diméthoxy-flavone, 5-hydroxy-8-méthoxy-flavone-7-O-D-glucuronide (ou wogonin-7-O-D-glucuronide ou oroxindine), 5-hydroxy-7,8-diméthoxy-flavone (ou 7-O-méthyl-wogonine), 5,7-dihydroxy-6-méthoxy-flavone (ou oroxyline A ou 6-O-méthyl-baïcaléine), 4′,5,7-trihydroxy-8-méthoxy-flavone (ou 4′-hydroxy-wogonine), 2′,5,6′-trihydroxy-7,8-diméthoxy-flavone, 5,7,8-trihydroxy-flavone (ou norwogonine), 5,6,7-trihydroxy-flavone (ou baïcaléine), 5,8-dihydroxy-6,7-diméthoxy-flavone, 2′,3,5,6′,7-pentahydroxy-flavone, 4′,5,6-trihydroxy-flavone-7-O-D-glucuronide (ou 4′-hydroxy-baïcaline), acide 5,6-dihydroxy-flavone-7-O-D-glucuronique méthyl ester (ou baïcaline méthyl ester), 2′,5,7-trihydroxy-flavone (2′-hydroxy chrysine), 5,7-dihydroxy-8-méthoxy-flavone (ou wogonine), 2′,5,7-trihydroxy-6′,8-diméthoxy-flavone (ou 2′-hydroxy-6′-méthoxy-wogonine), 4′,5,6,7-tétrahydroxy-flavone (ou 4′-hydroxy-baïcaléine), 5,6-dihydroxy-flavone-7-O-D-glucoside (baïcaléine-7-O-D-glucoside), 5-hydroxy-4′,6,7-triméthoxy-flavone (ou salvigénine), 5-hydroxy-6-méthoxy-flavone-7-O-D-glucuronide (ou oroxyline A-7-O-D-glucuronide), 5,6-dihydroxy-flavone-7-O-D-glucuronide (ou baïcaline), acide 5-hydroxy-6-méthoxy-flavone-7-O-D-glucuronique méthyl ester (ou oroxindine méthyl ester), 5,7-dihydroxy-flavone (ou chrysine).

10. Composition cosmétique ou pharmaceutique selon la revendication 9, caractérisée en ce que ladite substance active est choisie parmi le groupe constitué par la wogonine, la 2′-hydroxy-wogonine, la baïcaléine, la néobaïcaléine, ’oroxindine et la baïcaline.

11. Composition cosmétique ou pharmaceutique selon la revendication 9 ou 10 caractérisée en ce que l'extrait précité est obtenu par une extraction par solvant, de préférence choisi parmi le groupe consistant d'un solvant polaire, notamment une solution alcoolique ou hydroalcoolique, ou une solution éthérée, d'un solvant organique apolaire, comme le n-hexane, le benzène, ou avantageusement une combinaison des deux.

12. Composition cosmétique ou pharmaceutique selon l'une des revendications 9 à 11, caractérisée en ce que l'extrait précité seul ou ladite substance active isolée d'un tel extrait ou obtenue par synthèse chimique, est introduit seul ou en mélange avec d'autres substances actives compatibles dans la phase lipidique des phases lamellaires lipidiques hydratées ou des liposomes.

13. Composition cosmétique ou pharmaceutique selon l'une des revendications 9 à 12, caractérisée en ce que l'extrait précité ou la substance active isolée d'un tel extrait ou obtenue par synthèse chimique est introduit seul ou en mélange avec d'autres substances actives compatibles dans la phase aqueuse des phases lamellaires lipidiques hydratées ou des liposomes.

14. Composition cosmétique ou pharmaceutique selon les revendications 9 à 13, caractérisée en ce que le Scutellaria est choisi parmi le groupe consistant de Scutellaria Baicalensis, Scutellaria Viscidula ou de Scutellaria Galericulata.

15. Composition cosmétique ou pharmaceutique selon les revendications 9 à 14, caractérisée en ce que l'extrait de Scutellaria est un extrait de racines de Scutellaria Baicalensis Georgi encore appelé extrait d'Ogon.

16. Composition pharmaceutique, notamment dermatologique, ou cosmétique, selon l'une des revendications 9 à 15, caractérisée en ce que la proportion en poids d'extrait de Scutellaria, exprimée en extrait sec ou de la substance active isolée d'un tel extrait ou obtenue par synthèse chimique précitée, est comprise entre 0,0001 et 2 %, de préférence entre 0,001 et 0,4 % en poids par rapport au poids total de la composition.

## Revendications pour l'Etat contractant suivant: ES

1. Procédé de préparation d'une composition à base de phases lamellaires lipidiques hydratées ou de liposomes, caractérisé en ce qu'on incorpore au moins en partie un extrait de Scutellaria, ou au moins une substance active isolée d'un tel extrait ou obtenue par synthèse chimique, choisie parmi : 2′,5,7-trihydroxy-8-méthoxy flavone (ou 2′-hydroxy-wogonine), 2′,5-dihydroxy-6,6′,7,8-tétraméthoxy-flavone (ou skullcap flavone II ou néobaïcaléine), 2′,5,5′,7-tétrahydroxy-6′,8-diméthoxy-flavone, 5-hydroxy-8-méthoxy-flavone-7-O-D-glucuronide (ou wogonin-7-O-D-glucuronide ou oroxindine), 5-hydroxy-7,8-diméthoxy-flavone (ou 7-O-méthyl-wogonine), 5,7-dihydroxy-6-méthoxy-flavone (ou oroxyline A ou 6-O-méthyl-baïcaléine), 4′,5,7′-trihydroxy-8-méthoxy-flavone (ou 4′-hydroxy-wogonine), 2′,3,5,6′-trihydroxy-7,8-diméthoxy-flavone, 5,7,8-trihydroxyflavone (ou norwogonine), 5,6,7-trihydroxy-flavone (ou baïcalèine), 5,8-dihydroxy-6,7-diméthoxy-flavone, 2′,3,5,6′,7-pentahydroxy-flavone, 4′,5,6-trihydroxy-flavone-7-O-D- glucuronide (ou 4′-hydroxy-baïcaline), acide 5,6-dihydroxy-flavone-7-O-D-glucuronique méthyl ester (ou baïcaline méthyl ester), 2′,5,7-trihydroxy-flavone (2′-hydroxy chrysine), 5,7-dihydroxy-8-méthoxy-flavone (ou wogonine), 2′,5,7-trihydroxy-6′,8-diméthoxy-flavone (ou 2′-hydroxy-6′-méthoxy-wogonine), 4′,5,6,7-tétrahydroxy-flavone (ou 4′-hydroxy-baïcaléine), 5,6-dihydroxy-flavone-7-O-D-glucoside (baïcaléine-7-O-D-glucoside), 5-hydroxy-4′,6,7-triméthoxy-flavone (ou salvigénine), 5-hydroxy-6-méthoxy-flavone-7-O-D-glucuronide (ou oroxyline A -7-O-D-glucuronide), 5,6-dihydroxy-flavone-7-O-D-glucuronide (ou baïcaline), acide 5-hydroxy-6-méthoxy-flavone-7-O-D-glucuronique méthyl ester (ou oroxindine méthyl ester), 5,7-dihydroxy-flavone (ou chrysine) ; dans lesdites phases lamellaires lipidiques hydratées ou lesdits liposomes.

2. Procédé selon la revendication 1, caractérisé en ce que ladite substance active est choisie parmi le groupe constitué par la wogonine, la 2′-hydroxy-wogonine, la baïcaléine, la nèobaïcaléine, l'oroxindine et la baïcaline.

3. Procédé selon la revendication 1, caractérisé en ce que l'extrait précité est obtenu par une extraction par solvant, de préférence choisi parmi le groupe consistant d'un solvant polaire, notamment une solution alcoolique ou hydroalcoolique, ou une solution éthérée, d'un solvant organique apolaire, comme le n-hexane, le benzène, ou avantageusement une combinaison des deux.

4. Procédé selon l'une des revendications 1 à 3, caractérisé en ce que l'extrait précité seul ou la substance active isolée d'un tel extrait ou obtenue par synthèse chimique d'un tel extrait, est utilisé seul ou en mélange avec d'autres substances actives compatibles.

5. Procédé selon l'une des revendications 1 à 4, caractérisé en ce que l'extrait précité seul ou ladite substance active isolée d'un tel extrait ou obtenue par synthèse chimique est introduit dans la phase lipidique des phases lamellaires lipidiques hydratées ou des liposomes.

6. Procédé selon l'une des revendications 1 à 4, caractérisé en ce que l'extrait précité ou la substance active isolée d'un tel extrait ou obtenue par synthèse chimique précitée est introduit dans la phase aqueuse des phases lamellaires lipidiques hydratées ou des liposomes.

7. Procédé selon l'une quelconque des revendications 1 à 6, caractérisé en ce que le Scutellaria est choisi parmi le groupe consistant de Scutellaria Baicalensis, de Scutellaria Viscidula ou de Scutellaria Galericulata.

8. Procédé selon l'une des revendications 1 à 7, caractérisé en ce que l'extrait de Scutellaria est un extrait de racines de Scutellaria Baicalensis Georgi encore appelé extrait d'Ogon.

9. Procédé de préparation d'une composition cosmétique ou pharmaceutique, notamment dermatologique, à activité anti-inflammatoire, anti-allergique ou anti-vieillissement, caractérisé en ce qu'on incorpore une composition à base de phases lamellaires lipidiques hydratées ou de liposomes contenant au moins en partie un extrait de Scutellaria, ou au moins une substance active isolée d'un tel extrait ou obtenue par synthèse chimique, choisie parmi : 2',5,7-trihydroxy-8-méthoxy-flavone (ou 2'-hydroxy-wogonine), 2',5-dihydroxy-6,6',7,8-tétraméthoxy-flavone (ou skullcap flavone II ou néobaïcaléine), 2',5,5',7-tétrahydroxy-6',8-diméthoxy-flavone, 5-hydroxy-8-méthoxy-flavone-7-O-D-glucuronide (ou wogonin-7-O-D-glucuronide ou oroxindine), 5-hydroxy-7,8-diméthoxy-flavone (ou 7-O-méthyl-wogonine), 5,7-dihydroxy-6-méthoxy-flavone (ou oroxyline A ou 6-O-méthyl-baïcaléine), 4',5,7-trihydroxy-8-méthoxy-flavone (ou 4'-hydroxy-wogonine), 2',5,6'-trihydroxy-7,8-diméthoxy-flavone, 5,7,8-trihydroxy-flavone (ou norwogonine), 5,6,7-trihydroxy-flavone (oubaïcaléine), 5,8-dihydroxy-6,7-diméthoxy-flavone, 2',3,5,6',7-pentahydroxy-flavone, 4',5,6-trihydroxy-flavone-7-O-D-glucuronide (ou 4'-hydroxy-baïcaline), acide 5,6-dihydroxy-flavone-7-O-D-glucuronique méthyl ester (ou baïcaline méthyl ester), 2',5,7-trihydroxy-flavone (2'-hydroxy chrysine), 5,7-dihydroxy-8-méthoxy-flavone (ou wogonine), 2',5,7-trihydroxy-6',8-diméthoxy-flavone (ou 2'-hydroxy-6'-méthoxy-wogonine), 4',5,6,7-tétrahydroxy-flavone (ou 4'-hydroxy-baïcaléine), 5,6-dihydroxy-flavone-7-O-D-glucoside (baïcaléine-7-O-D-glucoside), 5-hydroxy-4',6,7-triméthoxy-flavone (ou salvigénine), 5-hydroxy-6-méthoxy-flavone-7-O-D-glucuronide (ou oroxyline A-7-O-D-glucuronide), 5,6-dihydroxy-flavone-7-O-D-glucuronide (ou baïcaline), acide 5-hydroxy-6-méthoxy-flavone-7-O-D-glucuronique méthyl ester (ou oroxindine méthyl ester), 5,7-dihydroxy-flavone (ou chrysine), préalablement préparée, dans un excipient ou support cosmétiquement ou pharmaceutiquement acceptable.

10. Procédé selon la revendication 9, caractérisé en ce que ladite substance active est choisie parmi le groupe constitué par la wogonine, la 2'-hydroxywogonine, la baïcaléine, la néobaïcaléine, l'oroxindine et la baïcaline.

11. Procédé selon la revendication 9 ou 10 caractérisé en ce que l'extrait précité est obtenu par une extraction par solvant, de préférence choisi parmi le groupe consistant d'un solvant polaire, notamment une solution alcoolique ou hydroalcoolique, ou une solution éthérée, d'un solvant organique apolaire, comme le n-hexane, le benzène, ou avantageusement une combinaison des deux.

12. Procédé selon l'une des revendications 9 à 11, caractérisé en ce que l'extrait précité seul ou ladite substance active isolée d'un tel extrait ou obtenue par synthèse chimique, est introduit seul ou en mélange avec d'autres substances actives compatibles dans la phase lipidique des phases lamellaires lipidiques hydratées ou des liposomes.

13. Procédé selon l'une des revendications 9 à 12, caractérisé en ce que l'extrait précité ou la substance active isolée d'un tel extrait ou obtenue par synthèse chimique est introduit seul ou en mélange avec d'autres substances actives compatibles dans la phase aqueuse des phases lamellaires lipidiques hydratées ou des liposomes.

14. Procédé selon les revendications 9 à 13, caractérisé en ce que le Scutellaria est choisi parmi le groupe consistant de Scutellaria Baicalensis, Scutellaria Viscidula ou de Scutellaria Galericulata.

15. Procédé selon l'une quelconque des revendications 9 à 14, caractérisé en ce que l'extrait de Scutellaria est un extrait de racines de Scutellaria Baicalensis Georgi encore appelé extrait d'Ogon.

16. Procédé selon l'une des revendications 9 à 15, caractérisé en ce que la proportion en poids d'extrait de Scutellaria, exprimée en extrait sec ou de la substance active isolée d'un tel extrait ou obtenue par synthèse chimique précitée, est comprise entre 0,0001 et 2 %, de préférence entre 0,001 et 0,4 % en poids par rapport au poids total de la composition.

## Revendications pour l'Etat contractant suivant: GR

1. Composition à base de phases lamellaires lipidiques hydratées ou de liposomes, caractérisée en ce que lesdites phases lamellaires lipidiques hydratées ou lesdits liposomes contiennent au moins en partie un extrait de Scutellaria, ou au moins une substance active isolée d'un tel extrait ou obtenue par synthèse chimique, choisie parmi : 2′,5,7-trihydroxy-8-méthoxy flavone (ou 2′-hydroxy-wogonine), 2′,5-dihydroxy-6,6′,7,8-tétraméthoxy-flavone (ou skullcap flavone II ou néobaïcaléine), 2′,5,5′,7-tétrahydroxy-6′,8-diméthoxy-flavone, 5-hydroxy-8-méthoxy-fllavone-7-O-D-glucuronide (ou wogonin-7-O-D-glucuronide ou oroxindine), 5-hydroxy-7,8-diméthoxy-flavone (ou 7-O-méthyl-wogonine), 5,7-dihydroxy-6-méthoxy-flavone (ou oroxyline A ou 6-O-méthyl-baïcaléine), 4′,5,7-trihydroxy-8-méthoxy-flavone (ou 4′-hydroxy-wogonine), 2′,5,6′-trihydroxy-7,8-diméthoxy-flavone, 5,7,8-trihydroxy-flavone (ou norwogonine), 5,6,7-trihydroxy-flavone (ou baïcaléine), 5,8-dihydroxy-6,7-diméthoxy-flavone, 2′,3,5,6′,7-pentahydroxy-flavone, 4′,5,6-trihydroxy-flavone-7-O-D-glucuronide (ou 4′-hydroxy-baïcaline ), acide 5,6-dihydroxy-flavone-7-O-D-glucuronique méthyl ester (ou baïcaline méthyl ester), 2′,5,7-trihydroxy-flavone (2′-hydroxy chrysine), 5,7-dihydroxy-8-méthoxy-flavone (ou wogonine), 2′,5,7-trihydroxy-6′,8-diméthoxy-flavone (ou 2′-hydroxy-6′-méthoxy-wogonine), 4′,5,6,7-tétrahydroxy-flavone (ou 4′-hydroxy-baïcaléine), 5,6-dihydroxy-flavone-7-O-D-glucoside (baïcaléine-7-O-D-glucoside), 5-hydroxy-4′,6,7-triméthoxy-flavone (ou salvigénine), 5-hydroxy-6-méthoxy-flavone-7-O-D-glucuronide (ou oroxyline A-7-O-D-glucuronide), 5,6-dihydroxy-flavone-7-O-D-glucuronide (ou baïcaline), acide 5-hydroxy-6-méthoxy-flavone-7-O-D-glucuronique méthyl ester (ou oroxindine méthyl ester), 5,7-dihydroxy-flavone (ou chrysine).

2. Composition selon la revendication 1, caractérisée en ce que ladite substance active est choisie parmi le groupe constitué par la wogonine, la 2′-hydroxy-wogonine, la baïcaléine, la néobaïcaléine, l'oroxindine et la baïcaline.

3. Composition selon la revendication 1, caractérisée en ce que l'extrait précité est obtenu par une extraction par solvant, de préférence choisi parmi le groupe consistant d'un solvant polaire, notamment une solution alcoolique ou hydroalcoolique, ou une solution éthérée, d'un solvant organique apolaire, comme le n-hexane, le benzène, ou avantageusement une combinaison des deux.

4. Composition selon l'une des revendications 1 à 3, caractérisée en ce que l'extrait précité seul ou la substance active isolée d'un tel extrait ou obtenue par synthèse chimique d'un tel extrait, est utilisé seul ou en mélange avec d'autres substances actives compatibles.

5. Composition selon l'une des revendications 1 à 4, caractérisée en ce que l'extrait précité seul ou ladite substance active isolée d'un tel extrait ou obtenue par synthèse chimique est introduit dans la phase lipidique des phases lamellaires lipidiques hydratées ou des liposomes.

6. Composition selon l'une des revendications 1 à 4, caractérisée en ce que l'extrait précité ou la substance active isolée d'un tel extrait ou obtenue par synthèse chimique précitée est introduit dans la phase aqueuse des phases lamellaires lipidiques hydratées ou des liposomes.

7. Composition selon l'une quelconque des revendications 1 à 6, caractérisée en ce que le Scutellaria est choisi parmi le groupe consistant de Scutellaria Baicalensis, de Scutellaria Viscidula ou de Scutellaria Galericulata.

8. Composition selon l'une des revendications 1 à 7, caractérisée en ce que l'extrait de Scutellaria est un extrait de racines de Scutellaria Baicalensis Georgi encore appelé extrait d'Ogon.

9. Composition cosmétique, caractérisée en ce qu'elle comprend une composition à base de phases lamellaires lipidiques hydratées ou de liposomes contenant au moins en partie un extrait de Scutellaria, ou au moins une substance active isolée d'un tel extrait ou obtenue par synthèse chimique, choisie parmi : 2′,5,7-trihydroxy-8-méthoxy-flavone (ou 2′-hydroxy-wogonine), 2′,5-dihydroxy-6,6′,7,8-tétraméthoxy-flavone (ou skullcap flavone II ou néobaïcaléine), 2′,5,5′,7-tétrahydroxy-6′,8-diméthoxy-flavone, 5-hydroxy-8-méthoxy-flavone-7-O-D-glucuronide (ou wogonin-7-O-D-glucuronide ou oroxindine), 5-hydroxy-7,8-diméthoxy-flavone (ou 7-O-méthyl-wogonine), 5,7-dihydroxy-6-méthoxy-flavone (ou oroxyline A ou 6-O-méthyl-baïcaléine), 4′,5,7-trihydroxy-8-méthoxy-flavone (ou 4′-hydroxy-wogonine), 2′,5,6′-trihydroxy-7,8-diméthoxy-flavone, 5,7,8-trihydroxy-flavone (ou norwogonine), 5,6,7-trihydroxy-flavone (oubaïcaléine), 5,8-dihydroxy-6,7-diméthoxy-flavone, 2′,3,5,6′,7-pentahydroxy-flavone, 4′,5,6-trihydroxy-flavone-7-O-D-glucuronide (ou 4′-hydroxy-baïcaline), acide 5,6-dihydroxy-flavone-7-O-D-glucuronique méthyl ester (ou baïcaline méthyl ester), 2′,5,7-trihydroxy-flavone (2′-hydroxy chrysine), 5,7-dihydroxy-8-méthoxy-flavone (ou wogonine), 2′,5,7-trihydroxy-6′,8-diméthoxy-flavone (ou 2′-hydroxy-6′-méthoxy-wogonine), 4′,5,6,7-tétrahydroxy-flavone (ou 4′-hydroxy-baïcaléine), 5,6-dihydroxy-flavone-7-O-D-glucoside (baïcaléine-7-O-D-glucoside), 5-hydroxy-4′,6,7-triméthoxy-flavone (ou salvigénine), 5-hydroxy-6-méthoxy-flavone-7-O-D-glucuronide (ou oroxyline A-7-O-D-glucuronide), 5,6-dihydroxy-flavone-7-O-D-glucuronide (ou baïcaline), acide 5-hydroxy-6-méthoxy-flavone-7-O-D-glucuronique méthyl ester (ou oroxindine méthyl ester), 5,7-dihydroxy-flavone (ou chrysine).

10. Composition cosmétique selon la revendication 9, caractérisée en ce que ladite substance active est choisie parmi le groupe constitué par la wogonine, la 2′-hydroxywogonine, la baïcaléine, la néobaïcaléine, l'oroxindine et la baïcaline.

11. Composition cosmétique selon la revendication 9 ou 10 caractérisée en ce que l'extrait précité est obtenu par une extraction par solvant, de préférence choisi parmi le groupe consistant d'un solvant polaire, notamment une solution alcoolique ou hydroalcoolique, ou une solution éthérée, d'un solvant organique apolaire, comme le n-hexane, le benzène, ou avantageusement une combinaison des deux.

12. Composition cosmétique selon l'une des revendications 9 à 11, caractérisée en ce que l'extrait précité seul ou ladite substance active isolée d'un tel extrait ou obtenue par synthèse chimique, est introduit seul ou en mélange avec d'autres substances actives compatibles dans la phase lipidique des phases lamellaires lipidiques hydratées ou des liposomes.

13. Composition cosmétique selon l'une des revendications 9 à 12, caractérisée en ce que l'extrait précité ou la substance active isolée d'un tel extrait ou obtenue par synthèse chimique est introduit seul ou en mélange avec d'autres substances actives compatibles dans la phase aqueuse des phases lamellaires lipidiques hydratées ou des liposomes.

14. Composition cosmétique selon les revendications 9 à 13, caractérisée en ce que le Scutellaria est choisi parmi le groupe consistant de Scutellaria Baicalensis, Scutellaria Viscidula ou de Scutellaria Galericulata.

15. Composition cosmétique selon les revendications 9 à 14, caractérisée en ce que l'extrait de Scutellaria est un extrait de racines de Scutellaria Baicalensis Georgi encore appelé extrait d'Ogon.

16. Composition cosmétique , selon l'une des revendications 9 à 15, caractérisée en ce que la proportion en poids d'extrait de Scutellaria, exprimée en extrait sec ou de la substance active isolée d'un tel extrait ou obtenue par synthèse chimique précitée, est comprise entre 0,0001 et 2 %, de préférence entre 0,001 et 0,4 % en poids par rapport au poids total de la composition.

17. Procédé de préparation d'une composition cosmétique ou pharmaceutique, à activité anti-inflammatoire, anti-allergique ou anti-vieillissement, caractérisée en ce qu'on incorpore une composition à base de phases lamellaires lipidiques hydratées ou de liposomes contenant au moins en partie un extrait de Scutellaria, ou au moins une substance active isolée d'un tel extrait ou obtenue par synthèse chimique, choisie parmi : 2′,5,7-trihydroxy-8-méthoxy-flavone (ou 2′-hydroxy-wogonine), 2′,5-dihydroxy-6,6′,7,8-tétraméthoxy-flavone (ou skullcap flavone II ou néobaïcaléine), 2′,5,5′,7-tétrahydroxy-6′,8-diméthoxy-flavone, 5-hydroxy-8-méthoxy-flavone-7-O-D-glucuronide (ou wogonin-7-O-D-glucuronide ou oroxindine), 5-hydroxy-7,8-diméthoxy-flavone (ou 7-O-méthyl-wogonine), 5,7-dihydroxy-6-méthoxy-flavone (ou oroxyline A ou 6-O-méthyl-baïcaléine), 4′,5,7-trihydroxy-8-méthoxy-flavone (ou 4′-hydroxy-wogonine), 2′,5,6′-trihydroxy-7,8-diméthoxy-flavone, 5,7,8-trihydroxy-flavone (ou norwogonine), 5,6,7-trihydroxy-flavone (oubaïcaléine), 5,8-dihydroxy-6,7-diméthoxy-flavone, 2′,3,5,6′,7-pentahydroxy-flavone, 4′,5,6-trihydroxy-flavone-7-O-D-glucuronide (ou 4′-hydroxy-baïcaline), acide 5,6-dihydroxy-flavone-7-O-D-glucuronique méthyl ester (ou baïcaline méthyl ester), 2′,5,7-trihydroxy-flavone (2′-hydroxy chrysine), 5,7-dihydroxy-8-méthoxy-flavone (ou wogonine), 2′,5,7-trihydroxy-6′,8-diméthoxy-flavone (ou 2′-hydroxy-6′-méthoxy-wogonine), 4′,5,6,7-tétrahydroxy-flavone (ou 4′-hydroxy-baïcaléine), 5,6-dihydroxy-flavone-7-O-D-glucoside (baïcaléine-7-O-D-glucoside), 5-hydroxy-4′,6,7-triméthoxy-flavone (ou salvigénine), 5-hydroxy-6-méthoxy-flavone-7-O-D-glucuronide (ou oroxyline A-7-O-D-glucuronide), 5,6-dihydroxy-flavone-7-O-D-glucuronide (ou baïcaline), acide 5-hydroxy-6-méthoxy-flavone-7-O-D-glucuronique méthyl ester (ou oroxindine méthyl ester), 5,7-dihydroxy-flavone (ou chrysine), préalablement préparée, dans un excipient ou support cosmétiquement ou pharmaceutiquement acceptable.


**Patentansprüche**


**Patentansprüche für folgende Vertragsstaaten : DE, GB, FR, IT, NL, SE, CH, LI, BE, AT, LU**

1. Zusammensetzung auf Basis von wasserhaltigen lamellaren Lipidphasen oder von Liposomen, dadurch gekennzeichnet, daß die wasserhaltigen lamellaren Lipidphasen bzw. die Liposome zumindest zum Teil einen Scutellaria-Extrakt oder zumindest eine aus einem solchen Extrakt isolierte oder durch chemische Synthese erhaltene aktive Substanz enthalten, ausgewählt aus: 2′,5,7-Trihydroxy-8-methoxyflavon (oder 2′-Hydroxywogonin), 2′,5-Dihydroxy-6,6′,7,8-tetramethoxyflavon (oder Helmkraut-Flavon II oder Neobaicalein), 2′,5,5′,7-Tetrahydroxy-6′,8-dimethoxyflavon, 5-Hydroxy-8-methoxyflavon-7-O-D-glucuronid (oder Wogonin-7-O-D-glucuronid oder Oroxindin), 5-Hydroxy-7,8-dimethoxyflavon (oder 7-O-Methylwogonin), 5,7-Dihydroxy-6-methoxyflavon (oder Oroxylin A oder 6-O-Methylbaicalein), 4′,5,7-Trihydroxy-8-methoxyflavon (oder 4′-Hydroxywogonin), 2′,5,6′-Trihydroxy-7,8-dimethoxyflavon, 5,7,8-Trihydroxy-flavon (oder Norwogonin), 5,6,7-Trihydroxyflavon (oder Baicalein), 5,8-Dihydroxy-6,7-dimethoxyflavon, 2′,3,5,6′,7-Pentahydroxyflavon, 4′,5,6-Trihydroxyflavon-7-O-D-glucuronid (oder 4′-Hydroxybaicalin), 5,6-Dihydroxyflavon-7-O-D-glucuronsäure-methyles-

EP 0 332 478 B1

ter (oder Baicalinmethylester), 2′,5,7-Trihydroxyflavon (2′-Hydroxychrysin), 5,7-Dihydroxy-8-methoxyflavon (oder Wogonin), 2′,5,7-Trihydroxy-6′,8-dimethoxyflavon (oder 2′-Hydroxy-6′-methoxywogonin), 4′,5,6,7-Tetrahydroxyflavon (oder 4′-Hydroxybaicalein), 5,6-dihydroxyflavon-7-O-D-glucosid (Baicalein-7-O-D-glucosid), 5-Hydroxy-4′,6,7-trimethoxyflavon (oder Salvigenin), 5-Hydroxy-6-methoxyflavon-7-O-D-glucuronid (oder Oroxylin A-7-O-D-glucuronid), 5,6-Dihydroxyflavon-7-O-D-glucuronid (oder Baicalin), 5-Hydroxy-6-methoxyflavon-7-O-D-glucuronsäure-methylester (oder Oroxindinmethylester), 5,7-Dihydroxyflavon (oder Chrysin).

2. Zusammensetzung nach Anspruch 1, dadurch gekennzeichnet, daß die aktive Substanz ausgewählt ist aus der Gruppe bestehend aus Wogonin, 2′-Hydroxywogonin, Baicalein, Neobaicalein, Oroxindin und Baicalin.

3. Zusammensetzung nach Anspruch 1, dadurch gekennzeichnet, daß der Extrakt durch Extraktion mit einem Lösungsmittel, vorzugsweise ausgewählt aus der Gruppe bestehend aus einem polaren Lösungsmittel, insbesondere einer alkoholischen oder wässerig-alkoholischen Lösung, oder einer etherischen Lösung, einem apolaren organischen Lösungsmittel, wie n-Hexan, Benzol, oder vorteilhafterweise einer Kombination aus beiden, erhalten ist.

4. Zusammensetzung nach einem der Ansprüche 1 bis 3, dadurch gekennzeichnet, daß der Extrakt allein oder die aus einem solchen Extrakt isolierte oder durch chemische Synthese eines solchen Extraktes erhaltene aktive Substanz allein oder in Mischung mit anderen verträglichen aktiven Substanzen verwendet wird.

5. Zusammensetzung nach einem der Ansprüche 1 bis 4, dadurch gekennzeichnet, daß der Extrakt allein oder die aus einem solchen Extrakt isolierte oder durch chemische Synthese erhaltene aktive Substanz in die Lipidphase der wasserhaltigen lamellaren Lipidphasen oder der Liposome eingebracht ist.

6. Zusammensetzung nach einem der Ansprüche 1 bis 4, dadurch gekennzeichnet, daß der Extrakt oder die aus einem solchen Extrakt isolierte oder durch chemische Synthese erhaltene aktive Substanz in die wässerige Phase der wasserhaltigen lamellaren Lipid-phasen oder der Liposome eingebracht ist.

7. Zusammensetzung nach einem der Ansprüche 1 bis 6, dadurch gekennzeichnet, daß Scutellaria ausgewählt ist aus der Gruppe bestehed aus Scutellaria Baicalensis, Scutellaria Viscidula oder Scutellaria Galericulata.

8. Zusammensetzung nach einem der Ansprüche 1 bis 7, dadurch gekennzeichnet, daß der Scutellaria-Extrakt ein Extrakt aus Wurzeln von Scutellaria Baicalensis Georgi, auch Ogon-Extrakt genannt, ist.

9. Kosmetische oder pharmazeutische, insbesondere dermatologische, Zusammensetzung mit entzündungshemmender, antiallergischer oder das Altern verhindernder Aktivität, dadurch gekennzeichnet, daß sie eine Zusammensetzung auf Basis von wasserhaltigen lamellaren Lipidphasen oder von Liposomen umfaßt, enthaltend zumindest zum Teil einen Scutellaria-Extrakt oder zumindest eine aus einem solchen Extrakt isolierte oder durch chemische Synthese erhaltene aktive Substanz, ausgewählt aus: 2′,5,7-Trihydroxy-8-methoxyflavon (oder 2′-Hydroxywogonin), 2′,5-Dihydroxy-6,6′,7,8-tetramethoxyflavon (oder Helmkraut-Flavon II oder Neobaicalein), 2′,5,5′,7-Tetrahydroxy-6′,8-dimethoxyflavon, 5-Hydroxy-8-methoxyflavon-7-O-D-glucuronid (oder Wogonin-7-O-D-glucuronid oder Oroxindin), 5-Hydroxy-7,8-dimethoxyflavon (oder 7-O-Methylwogonin), 5,7-Dihydroxy-6-methoxyflavon (oder Oroxylin A oder 6-O-Methylbaicalein), 4′,5,7-Trihydroxy-8-methoxyflavon (oder 4′-Hydroxywogonin), 2′,5,6′-Trihydroxy-7,8-dimethoxyflavon, 5,7,8-Trihydroxy-flavon (oder Norwogonin), 5,6,7-Trihydroxyflavon (oder Baicalein), 5,8-Dihydroxy-6,7-dimethoxyflavon, 2′,3,5,6′,7-Pentahydroxyflavon, 4′,5,6-Trihydroxyflavon-7-O-D-glucuronid (oder 4′-Hydroxybaicalin), 5,6-Dihydroxyflavon-7-O-D-glucuronsäure-methylester (oder Baicalinmethylester), 2′,5,7-Trihydroxyflavon (2′-Hydroxychrysin), 5,7-Dihydroxy-8-methoxyflavon (oder Wogonin), 2′,5,7-Trihydroxy-6′,8-dimethoxyflavon (oder 2′-Hydroxy-6′-methoxywogonin), 4′,5,6,7-Tetrahydroxyflavon (oder 4′-Hydroxybaicalein), 5,6-dihydroxyflavon-7-O-D-glucosid (Baicalein-7-O-D-glucosid), 5-Hydroxy-4′,6,7-trimethoxyflavon (oder Salvigenin), 5-Hydroxy-6-methoxyflavon-7-O-D-glucuronid (oder Oroxylin A-7-O-D-glucuronid), 5,6-Dihydroxyflavon-7-O-D-glucuronid (oder Baicalin), 5-Hydroxy-6-methoxyflavon-7-O-D-glucuronsäure-methylester (oder Oroxindin-methylester), 5,7-Dihydroxyflavon (oder Chrysin).

10. Kosmetische oder pharmazeutische Zusammensetzung nach Anspruch 9, dadurch gekennzeichnet, daß die aktive Substanz ausgewählt ist aus der Gruppe bestehend aus Wogonin, 2′-Hydroxywogonin, Baicalein, Neobaicalein, Oroxindin und Baicalin.

11. Kosmetische oder pharmazeutische Zusammensetzung nach Anspruch 9 oder 10, dadurch gekennzeichnet, daß der Extrakt durch Extraktion mit einem Lösungsmittel, vorzugsweise ausgewählt aus der Gruppe bestehend aus einem polaren Lösungsmittel, insbesondere einer alkoholischen oder wässerig-alkoholischen Lösung, oder einer etherischen Lösung, einem apolaren organischen Lösungsmittel, wie n-Hexan, Benzol, oder vorteilhafterweise einer Kombination aus beiden, erhalten ist.

12. Kosmetische oder pharmazeutische Zusammensetzung nach einem der Ansprüche 9 bis 11, dadurch gekennzeichnet, daß der Extrakt allein oder die aus einem solchen Extrakt isolierte oder durch chemische Synthese erhaltene aktive Substanz, allein oder in Mischung mit anderen verträglichen aktiven Substanzen, in die Lipidphase der wasserhaltigen lamellaren Lipidphasen oder der Liposome eingebracht ist.

18

13. Kosmetische oder pharmazeutische Zusammensetzung nach einem der Ansprüche 9 bis 12, dadurch gekennzeichnet, daß der Extrakt oder die aus einem solchen Extrakt isolierte oder durch chemische Synthese erhaltene aktive Substanz allein oder in Mischung mit anderen verträglichen aktiven Substanzen in die wässerige Phase der wasserhaltigen lamellaren Lipidphasen oder der Liposome eingebracht ist.

14. Kosmetische oder pharmazeutische Zusammensetzung nach den Ansprüchen 9 bis 13 , dadurch gekennzeichnet, daß Scutellaria ausgewählt ist aus der Gruppe bestehed aus Scutellaria Baicalensis, Scutellaria Viscidula oder Scutellaria Galericulata.

15. Kosmetische oder pharmazeutische Zusammensetzung nach einem der Ansprüche 9 bis 14, dadurch gekennzeichnet, daß der Scutellaria-Extrakt ein Extrakt aus Wurzeln von Scutellaria Baicalensis Georgi, auch Ogon-Extrakt genannt, ist.

16. Pharmazeutische, insbesondere dermatologische, oder kosmetische Zusammensetzung nach einem der Ansprüche 9 bis 15, dadurch gekennzeichnet, daß der Gewichtsanteil an Scutellaria-Extrakt, ausgedrückt als Trockenextrakt, oder an aus einem solchen Extrakt isolierter oder durch chemische Synthese erhaltener aktiver Substanz zwischen 0,0001 und 2 Gew.%, vorzugsweise zwischen 0,001 und 0,4 Gew.%, bezogen auf das Gesamtgewicht der Zusammensetzung beträgt.

**Patentansprüche für folgenden Vertragsstaat: ES**

1. Verfahren zur Herstellung einer Zusammensetzung auf Basis von wasserhaltigen lamellaren Lipidphasen oder von Liposomen, dadurch gekennzeichnet, daß zumindest zum Teil ein Scutellaria-Extrakt oder zumindest eine aus einem solchen Extrakt isolierte oder durch chemische Synthese erhaltene aktive Substanz, ausgewählt aus: 2′,5,7-Trihydroxy-8-methoxyflavon (oder 2′-Hydroxywogonin), 2′,5-Dihydroxy-6,6′,7,8-tetramethoxyflavon (oder Helmkraut-Flavon II oder Neobaicalein), 2′,5,5′,7-Tetrahydroxy-6′,8-dimethoxyflavon, 5-Hydroxy-8-methoxyflavon-7-O-D-glucuronid (oder Wogonin-7-O-D-glucuronid oder Oroxindin), 5-Hydroxy-7,8-dimethoxyflavon (oder 7-O-Methylwogonin), 5,7-Dihydroxy-6-methoxyflavon (oder Oroxylin A oder 6-O-Methylbaicalein), 4′,5,7-Trihydroxy-8-methoxyflavon (oder 4′-Hydroxywogonin), 2′,5,6′-Trihydroxy-7,8-dimethoxyflavon, 5,7,8-Trihydroxyflavon (oder Norwogonin), 5,6,7-Trihydroxyflavon (oder Baicalein), 5,8-Dihydroxy-6,7-dimethoxyflavon, 2′,3,5,6′,7-Pentahydroxyflavon, 4′,5,6-Trihydroxyflavon-7-O-D-glucuronid (oder 4′-Hydroxybaicalin), 5,6-Dihydroxyflavon-7-O-D-glucuronsäure-methylester (oder Baicalinmethylester), 2′,5,7-Trihydroxyflavon (2′-Hydroxychrysin), 5,7-Dihydroxy-8-methoxyflavon (oder Wogonin), 2′,5,7-Trihydroxy-6′,8-dimethoxyflavon (oder 2′-Hydroxy-6′-methoxywogonin), 4′,5,6,7-Tetrahydroxyflavon (oder 4′-Hydroxybaicalein), 5,6-dihydroxyflavon-7-O-D-glucosid (Baicalein-7-O-D-glucosid), 5-Hydroxy-4′,6,7-trimethoxyflavon (oder Salvigenin), 5-Hydroxy-6-methoxyflavon-7-O-D-glucuronid (oder Oroxylin A-7-O-D-glucuronid), 5,6-Dihydroxyflavon-7-O-D-glucuronid (oder Baicalin), 5-Hydroxy-6-methoxyflavon-7-O-D-glucuronsäure-methylester (oder Oroxindinmethylester), 5,7-Dihydroxyflavon (oder Chrysin), in die wasserhaltigen lamellaren Lipidphasen oder die Liposome eingearbeitet wird.

2. Verfahren nach Anspruch 1, dadurch gekennzeichnet, daß die aktive Substanz ausgewählt ist aus der Gruppe bestehend aus Wogonin, 2′-Hydroxywogonin, Baicalein, Neobaicalein, Oroxindin und Baicalin.

3. Verfahren nach Anspruch 1, dadurch gekennzeichnet, daß der Extrakt durch Extraktion mit einem Lösungsmittel, vorzugsweise ausgewählt aus der Gruppe bestehend aus einem polaren Lösungsmittel, insbesondere einer alkoholischen oder wässerig-alkoholischen Lösung, oder einer etherischen Lösung, einem apolaren organischen Lösungsmittel, wie n-Hexan, Benzol, oder vorteilhafterweise einer Kombination aus beiden, erhalten wird.

4. Verfahren nach einem der Ansprüche 1 bis 3, dadurch gekennzeichnet, daß der Extrakt allein oder die aus einem solchen Extrakt isolierte oder durch chemische Synthese eines solchen Extraktes erhaltene aktive Substanz allein oder in Mischung mit anderen verträglichen aktiven Substanzen verwendet wird.

5. Verfahren nach einem der Ansprüche 1 bis 4, dadurch gekennzeichnet, daß der Extrakt allein oder die aus einem solchen Extrakt isolierte oder durch chemische Synthese erhaltene aktive Substanz in die Lipidphase der wasserhaltigen lamellaren Lipidphasen oder der Liposome eingebracht wird.

6. Verfahren nach einem der Ansprüche 1 bis 4, dadurch gekennzeichnet, daß der Extrakt oder die aus einem solchen Extrakt isolierte oder durch chemische Synthese erhaltene aktive Substanz in die wässerige Phase der wasserhaltigen lamellaren Lipid-phasen oder der Liposome eingebracht wird.

7. Verfahren nach einem der Ansprüche 1 bis 6, dadurch gekennzeichnet, daß Scutellaria ausgewählt ist aus der Gruppe bestehed aus Scutellaria Baicalensis, Scutellaria Viscidula oder Scutellaria Galericulata.

8. Verfahren nach einem der Ansprüche 1 bis 7, dadurch gekennzeichnet, daß der Scutellaria-Extrakt ein Extrakt aus Wurzeln von Scutellaria Baicalensis Georgi, auch Ogon-Extrakt genannt, ist.

9. Verfahren zur Herstellung einer kosmetischen oder pharmazeutischen, insbesondere dermatologischen, Zusammensetzung mit entzündungshemmender, antiallergischer oder das Altern verhindernder Aktivität, dadurch gekennzeichnet, daß eine zuvor hergestellte Zusammensetzung auf Basis von wasserhaltigen lamellaren Lipidphasen oder von Liposomen, enthaltend zumindenst zum Teil einen Scutellaria-Extrakt oder zumindest eine aus einem solchen Extrakt isolierte oder durch chemische Synthese erhaltene aktive Substanz, ausgewählt aus: 2',5,7-Trihydroxy-8-methoxyflavon (oder 2'-Hydroxywogonin), 2',5-Dihydroxy-6,6',7,8-tetramethoxyflavon (oder Helmkraut-Flavon II oder Neobaicalein), 2',5,5',7-Tetrahydroxy-6',8-dimethoxyflavon, 5-Hydroxy-8-methoxyflavon-7-O-D-glucuronid (oder Wogonin-7-O-D-glucuronid oder Oroxindin), 5-Hydroxy-7,8-dimethoxyflavon (oder 7-O-Methylwogonin), 5,7-Dihydroxy-6-methoxyflavon (oder Oroxylin A oder 6-O-Methylbaicalein), 4',5,7-Trihydroxy-8-methoxyflavon (oder 4'-Hydroxywogonin), 2',5,6'-Trihydroxy-7,8-dimethoxyflavon, 5,7,8-Trihydroxyflavon (oder Norwogonin), 5,6,7-Trihydroxyflavon (oder Baicalein), 5,8-Dihydroxy-6,7-dimethoxyflavon, 2',3,5,6',7-Pentahydroxyflavon, 4',5,6-Trihydroxyflavon-7-O-D-glucuronid (oder 4'-Hydroxybaicalin), 5,6-Dihydroxyflavon-7-O-D-glucuronsäure-methylester (oder Baicalinmethylester), 2',5,7-Trihydroxyflavon (2'-Hydroxychrysin), 5,7-Dihydroxy-8-methoxyflavon (oder Wogonin), 2',5,7-Trihydroxy-6',8-dimethoxyflavon (oder 2'-Hydroxy-6'-methoxywogonin), 4',5,6,7-Tetrahydroxyflavon (oder 4'-Hydroxybaicalein), 5,6-dihydroxyflavon-7-O-D-glucosid (Baicalein-7-O-D-glucosid), 5-Hydroxy-4',6,7-trimethoxyflavon (oder Salvigenin), 5-Hydroxy-6-methoxyflavon-7-O-D-glucuronid (oder Oroxylin A-7-O-D-glucuronid), 5,6-Dihydroxyflavon-7-O-D-glucuronid (oder Baicalin), 5-Hydroxy-6-methoxyflavon-7-O-D-glucuronsäure-methylester (oder Oroxindinmethylester), 5,7-Dihydroxyflavon (oder Chrysin),in einen kosmetisch oder pharmazeutisch akzeptablen Exzipienten oder Träger eingearbeitet wird.

10. Verfahren nach Anspruch 9, dadurch gekennzeichnet, daß die aktive Substanz ausgewählt ist aus der Gruppe bestehend aus Wogonin, 2'-Hydroxywogonin, Baicalein, Neobaicalein, Oroxindin und Baicalin.

11. Verfahren nach Anspruch 9 oder 10, dadurch gekennzeichnet, daß der Extrakt durch Extraktion mit einem Lösungsmittel, vorzugsweise ausgewählt aus der Gruppe bestehend aus einem polaren Lösungsmittel, insbesondere einer alkoholischen oder wässerig-alkoholischen Lösung, oder einer etherischen Lösung, einem apolaren organischen Lösungsmittel, wie n-Hexan, Benzol, oder vorteilhafterweise einer Kombination aus beiden, erhalten wird.

12. Verfahren nach einem der Ansprüche 9 bis 11, dadurch gekennzeichnet, daß der Extrakt allein oder die aus einem solchen Extrakt isolierte oder durch chemische Synthese erhaltene aktive Substanz, allein oder in Mischung mit anderen verträglichen aktiven Substanzen, in die Lipidphase der wasserhaltigen lamellaren Lipidphasen oder der Liposome eingebracht wird.

13. Verfahren nach einem der Ansprüche 9 bis 12, dadurch gekennzeichnet, daß der Extrakt oder die aus einem solchen Extrakt isolierte oder durch chemische Synthese erhaltene aktive Substanz allein oder in Mischung mit anderen verträglichen aktiven Substanzen in die wässerige Phase der wasserhaltigen lamellaren Lipidphasen oder der Liposome eingebracht wird.

14. Verfahren nach den Ansprüchen 9 bis 13, dadurch gekennzeichnet, daß Scutellaria ausgewählt ist aus der Gruppe bestehed aus Scutellaria Baicalensis, Scutellaria Viscidula oder Scutellaria Galericulata.

15. Verfahren nach einem der Ansprüche 9 bis 14, dadurch gekennzeichnet, daß der Scutellaria-Extrakt ein Extrakt aus Wurzeln von Scutellaria Baicalensis Georgi, auch Ogon-Extrakt genannt, ist.

16. Verfahren nach einem der Ansprüche 9 bis 15 , dadurch gekennzeichnet, daß der Gewichtsanteil an Scutellaria-Extrakt, ausgedrückt als Trockenextrakt, oder an aus einem solchen Extrakt isolierter oder durch chemische Synthese erhaltener aktiver Substanz zwischen 0,0001 und 2 Gew.%, vorzugsweise zwischen 0,001 und 0,4 Gew.%, bezogen auf das Gesamtgewicht der Zusammensetzung, beträgt.

**Patentansprüche für folgenden Vertragsstaat: GR**

1. Zusammensetzung auf Basis von wasserhaltigen lamellaren Lipidphasen oder von Liposomen, dadurch gekennzeichnet, daß die wasserhaltigen lamellaren Lipidphasen bzw. die Liposome zumindest zum Teil einen Scutellaria-Extrakt oder zumindest eine aus einem solchen Extrakt isolierte oder durch chemische Synthese erhaltene aktive Substanz enthalten, ausgewählt aus: 2',5,7-Trihydroxy-8-methoxyflavon (oder 2'-Hydroxywogonin), 2',5-Dihydroxy-6,6',7,8-tetramethoxyflavon (oder Helmkraut-Flavon II oder Neobaicalein), 2',5,5',7-Tetrahydroxy-6',8-dimethoxyflavon, 5-Hydroxy-8-methoxyflavon-7-O-D-glucuronid (oder Wogonin-7-O-D-glucuronid oder Oroxindin), 5-Hydroxy-7,8-dimethoxyflavon (oder 7-O-Methylwogonin), 5,7-Dihydroxy-6-methoxyflavon (oder Oroxylin A oder 6-O-Methylbaicalein), 4',5,7-Trihydroxy-8-methoxyflavon (oder 4'-Hydroxywogonin), 2',5,6'-Trihydroxy-7,8-dimethoxyflavon, 5,7,8-Trihydroxyflavon (oder Norwogonin), 5,6,7-Trihydroxyflavon (oder Baicalein), 5,8-Dihydroxy-6,7-dimethoxyflavon, 2',3,5,6',7-Pentahydroxyflavon, 4',5,6-Trihydroxyflavon-7-O-D-glucuronid (oder 4'-Hydroxybaicalin), 5,6-Dihydroxyflavon-7-O-D-glucuronsäure-methylester (oder Baicalinmethylester), 2',5,7-Trihydroxyflavon (2'-Hydroxychrysin), 5,7-Dihydroxy-8-methoxyflavon (oder Wogonin), 2',5,7-Trihydroxy-6',8-dimethoxyflavon (oder 2'-Hydroxy-6'-methoxywogonin), 4',5,6,7-Tetrahydroxyflavon (oder 4'-Hydroxybaicalein), 5,6-dihydroxyflavon-7-O-D-glucosid (Baicalein-7-O-D-glucosid), 5-Hydroxy-4′,6,7-trimethoxyflavon (oder Salvigenin), 5-Hydroxy-6-methoxyflavon-7-O-D-glucuronid (oder Oroxylin A-7-O-D-glucuronid), 5,6-Dihydroxyflavon-7-O-D-glucuronid (oder Baicalin), 5-Hydroxy-6-methoxyflavon-7-O-D-glucuronsäure-methylester (oder Oroxindinmethylester), 5,7-Dihydroxyflavon (oder Chrysin).

2. Zusammensetzung nach Anspruch 1, dadurch gekennzeichnet, daß die aktive Substanz ausgewählt ist aus der Gruppe bestehend aus Wogonin, 2′-Hydroxywogonin, Baicalein, Neobaicalein, Oroxindin und Baicalin.

3. Zusammensetzung nach Anspruch 1, dadurch gekennzeichnet, daß der Extrakt durch Extraktion mit einem Lösungsmittel, vorzugsweise ausgewählt aus der Gruppe bestehend aus einem polaren Lösungsmittel, insbesondere einer alkoholischen oder wässerig-alkoholischen Lösung, oder einer etherischen Lösung, einem apolaren organischen Lösungsmittel, wie n-Hexan, Benzol, oder vorteilhafterweise einer Kombination aus beiden, erhalten ist.

4. Zusammensetzung nach einem der Ansprüche 1 bis 3, dadurch gekennzeichnet, daß der Extrakt allein oder die aus einem solchen Extrakt isolierte oder durch chemische Synthese eines solchen Extraktes erhaltene aktive Substanz allein oder in Mischung mit anderen verträglichen aktiven Substanzen verwendet wird.

5. Zusammensetzung nach einem der Ansprüche 1 bis 4, dadurch gekennzeichnet, daß der Extrakt allein oder die aus einem solchen Extrakt isolierte oder durch chemische Synthese erhaltene aktive Substanz in die Lipidphase der wasserhaltigen lamellaren Lipidphasen oder der Liposome eingebracht ist.

6. Zusammensetzung nach einem der Ansprüche 1 bis 4, dadurch gekennzeichnet, daß der Extrakt oder die aus einem solchen Extrakt isolierte oder durch chemische Synthese erhaltene aktive Substanz in die wässerige Phase der wasserhaltigen lamellaren Lipid-phasen oder der Liposome eingebracht ist.

7. Zusammensetzung nach einem der Ansprüche 1 bis 6, dadurch gekennzeichnet, daß Scutellaria ausgewählt ist aus der Gruppe bestehed aus Scutellaria Baicalensis, Scutellaria Viscidula oder Scutellaria Galericulata.

8. Zusammensetzung nach einem der Ansprüche 1 bis 7, dadurch gekennzeichnet, daß der Scutellaria-Extrakt ein Extrakt aus Wurzeln von Scutellaria Baicalensis Georgi, auch Ogon-Extrakt genannt, ist.

9. Kosmetische Zusammensetzung, dadurch gekennzeichnet, daß sie eine Zusammensetzung auf Basis von wasserhaltigen lamellaren Lipidphasen oder von Liposomen umfaßt, enthaltend zumindenst zum Teil einen Scutellaria-Extrakt oder zumindest eine aus einem solchen Extrakt isolierte oder durch chemische Synthese erhaltene aktive Substanz, ausgewählt aus: 2′,5,7-Trihydroxy-8-methoxyflavon (oder 2′-Hydroxywogonin), 2′,5-Dihydroxy-6,6′,7,8-tetramethoxyflavon (oder Helmkiaut-Flavon II oder Neobaicalein), 2′,5,5′,7-Tetrahydroxy-6′,8-dimethoxyflavon, 5-Hydroxy-8-methoxyflavon-7-O-D-glucuronid (oder Wogonin-7-O-D-glucuronid oder Oroxindin), 5-Hydroxy-7,8-dimethoxyflavon (oder 7-O-Methylwogonin), 5,7-Dihydroxy-6-methoxyflavon (oder Oroxylin A oder 6-O-Methylbaicalein), 4′,5,7-Trihydroxy-8-methoxyflavon (oder 4′-Hydroxywogonin), 2′,5,6′-Trihydroxy-7,8-dimethoxyflavon, 5,7,8-Trihydroxyflavon (oder Norwogonin), 5,6,7-Trihydroxyflavon (oder Baicalein), 5,8-Dihydroxy-6,7-dimethoxyflavon, 2′,3,5,6′,7-Pentahydroxyflavon, 4′,5,6-Trihydroxyflavon-7-O-D-glucuronid (oder 4′-Hydroxybaicalin), 5,6-Dihydroxyflavon-7-O-D-glucuronsäure-methylester (oder Baicalinmethylester), 2′,5,7-Trihydroxyflavon (2′-Hydroxychrysin), 5,7-Dihydroxy-8-methoxyflavon (oder Wogonin), 2′,5,7-Trihydroxy-6′,8-dimethoxyflavon (oder 2′-Hydroxy-6′-methoxywogonin), 4′,5,6,7-Tetrahydroxyflavon (oder 4′-Hydroxybaicalein), 5,6-dihydroxyflavon-7-O-D-glucosid (Baicalein-7-O-D-glucosid), 5-Hydroxy-4′,6,7-trimethoxyflavon (oder Salvigenin), 5-Hydroxy-6-methoxyflavon-7-O-D-glucuronid (oder Oroxylin A-7-O-D-glucuronid), 5,6-Dihydroxyflavon-7-O-D-glucuronid (oder Baicalin), 5-Hydroxy-6-methoxyflavon-7-O-D-glucuronsäure-methylester (oder Oroxindinmethylester), 5,7-Dihydroxyflavon (oder Chrysin).

10. Kosmetische Zusammensetzung nach Anspruch 9, dadurch gekennzeichnet, daß die aktive Substanz ausgewählt ist aus der Gruppe bestehend aus Wogonin, 2′-Hydroxywogonin, Baicalein, Neobaicalein, Oroxindin und Baicalin.

11. Kosmetische Zusammensetzung nach Anspruch 9 oder 10, dadurch gekennzeichnet, daß der Extrakt durch Extraktion mit einem Lösungsmittel, vorzugsweise ausgewählt aus der Gruppe bestehend aus einem polaren Lösungsmittel, insbesondere einer alkoholischen oder wässerig-alkoholischen Lösung, oder einer etherischen Lösung, einem apolaren organischen Lösungsmittel, wie n-Hexan, Benzol, oder vorteilhafterweise einer Kombination aus beiden, erhalten ist.

12. Kosmetische Zusammensetzung nach einem der Ansprüche 9 bis 11, dadurch gekennzeichnet, daß der Extrakt allein oder die aus einem solchen Extrakt isolierte oder durch chemische Synthese erhaltene aktive Substanz, allein oder in Mischung mit anderen verträglichen aktiven Substanzen, in die Lipidphase der wasserhaltigen lamellaren Lipidphasen oder der Liposome eingebracht ist.

13. Kosmetische Zusammensetzung nach einem der Ansprüche 9 bis 12, dadurch gekennzeichnet, daß der Extrakt oder die aus einem solchen Extrakt isolierte oder durch chemische Synthese erhaltene aktive Substanz allein oder in Mischung mit anderen verträglichen aktiven Substanzen in die wässerige Phase der wasserhaltigen lamellaren Lipidphasen oder der Liposome eingebracht ist.

14. Kosmetische Zusammensetzung nach den Ansprüchen 9 bis 13, dadurch gekennzeichnet, daß Scutellaria ausgewählt ist aus der Gruppe bestehed aus Scutellaria Baicalensis, Scutellaria Viscidula oder Scutellaria Galericulata.

15. Kosmetische Zusammensetzung nach einem der Ansprüche 9 bis 14, dadurch gekennzeichnet, daß der Scutellaria-Extrakt ein Extrakt aus Wurzeln von Scutellaria Baicalensis Georgi, auch Ogon-Extrakt genannt, ist.

16. Kosmetische Zusammensetzung nach einem der Ansprüche 9 bis 15, dadurch gekennzeichnet, daß der Gewichtsanteil an Scutellaria-Extrakt, ausgedrückt als Trockenextrakt, oder an aus einem solchen Extrakt isolierter oder durch chemische Synthese erhaltener aktiver Substanz zwischen 0,0001 und 2 Gew.%, vorzugsweise zwischen 0,001 und 0,4 Gew.%, bezogen auf das Gesamtgewicht der Zusammensetzung,beträgt.

17. Verfahren zur Herstellung einer kosmetischen oder pharmazeutischen Zusammensetzung mit entzündungshemmender, antiallergischer oder das Altern verhindernder Aktivität, dadurch gekennzeichnet, daß eine zuvor hergestellte Zusammensetzung auf Basis von wasserhaltigen lamellaren Lipidphasen oder von Liposomen, enthaltend zumindest zum Teil einen Scutellaria-Extrakt oder zumindest eine aus einem solchen Extrakt isolierte oder durch chemische Synthese erhaltene aktive Substanz, ausgewählt aus: 2',5,7-Trihydroxy-8-methoxyflavon (oder 2'-Hydroxywogonin), 2',5-Dihydroxy-6,6',7,8-tetramethoxyflavon (oder Helmkraut-Flavon II oder Neobaicalein), 2',5,5',7-Tetrahydroxy-6',8-dimethoxyflavon, 5-Hydroxy-8-methoxyflavon-7-O-D-glucuronid (oder Wogonin-7-O-D-glucuronid oder Oroxindin), 5-Hydroxy-7,8-dimethoxyflavon (oder 7-O-Methylwogonin), 5,7-Dihydroxy6-methoxyflavon (oder Oroxylin A oder 6-O-Methylbaicalein), 4',5,7-Trihydroxy-8-methoxyflavon (oder 4'-Hydroxwogonin), 2',5,6'-Trihydroxy-7,8-dimethoxyflavon, 5,7,8-Trihydroxyflavon (oder Norwogonin), 5,6,7-Trihydroxyflavon (oder Baicalein), 5,8-Dihydroxy-6,7-dimethoxyflavon, 2',3,5,6',7-Pentahydroxyflavon, 4',5,6-Trihydroxyflavon-7-O-D-glucuronid (oder 4'-Hydroxybaicalin), 5,6-Dihydroxyflavon-7-O-D-glucuronsäure-methylester (oder Baicalinmethylester), 2',5,7-Trihydroxyflavon (2'-Hydroxychrysin), 5,7-Dihydroxy-8-methoxyflavon (oder Wogonin), 2',5,7-Trihydroxy-6',8-dimethoxyflavon (oder 2'-Hydroxy-6'-methoxywogonin), 4',5,6,7-Tetrahydroxyflavon (oder 4'-Hydroxybaicalein), 5,6-dihydroxyflavon-7-O-D-glucosid (Baicalein-7-O-D-glucosid), 5-Hydroxy-4',6,7-trimethoxyflavon (oder Salvigenin), 5-Hydroxy-6-methoxyflavon-7-O-D-glucuronid (oder Oroxylin A-7-O-D-glucuronid), 5,6-Dihydroxyflavon-7-O-D-glucuronid (oder Baicalin), 5-Hydroxy-6-methoxyflavon-7-O-D-glucuronsäure-methylester (oder Oroxindinmethylester), 5,7-Dihydroxyflavon (oder Chrysin), in einen kosmetisch oder pharmazeutisch akzeptablen Exzipienten oder Träger eingearbeitet wird.

## Claims

### Claims for the following Contrating States : DE, GB, FR, IT, NL, SE, CH, LI, BE, AT, LU

1. Composition based on hydrated lipidic lamellar phases or liposomes, characterized in that said hydrated lipidic lamellar phases or said liposomes contain at least partly an extract of Scutellaria, or at least one active substance isolated from such an extract or obtained by chemical synthesis, selected from: 2',5,7-trihydroxy-8-methoxy flavone (or 2'-hydroxy-wogonine), 2'5-dihydroxy-(6,6',7,8-tetramethoxy-flavone (or skullcap flavone II or neobaicaleine), 2',5,5',7-tetrahydroxy-6',8-dimethoxy-flavone, 5-hydroxy-8-methoxy-flavone-7-O-D-glucuronide (or wogonine-7-O-D-glucuronide or oroxindine), 5-hydroxy-7,8-dimethoxy-flavone (or 7-O-methyl-wogonine), 5,7-dihydroxy-6-methoxy-flavone (or oroxyline A or 6-O-methyl-baicaleine), 4',5,7-trihydroxy-8-methoxy-flavone (or 4'-hydroxy-wogonine), 2',5,6'-trihydroxy-7,8-dimethoxy-flavone, 5,7,8-trihydroxy-flavone (or norwogonine), 5,6, 7-trihydroxy-flavone (or baicaleine), 5,8-dihydroxy-6,7-dimethoxy-flavone, 2',3,5,6',7-pentahydroxy-flavone, 4',5,6-trihydroxy-flavone-7-O-D-glucuronide (or 4'-hydroxy-baicaline), 5,6-dihydroxy-flavone-7-O-D-glucuronic methyl ester acid (or methyl ester baicaline), 2',5,7-trihydroxy-flavone (2'-hydroxy chrysine), 5,7-dihydroxy-8-methoxy-flavone (or wogonine), 2',5,7-trihydroxy-6',8-dimethoxy-flavone (or 2'-hydroxy-6'-methoxy-wogonine), 4',5,6,7-tetrahydroxy-flavone (or 4'-hydroxy-baicaleine), 5,6-dihydroxy-flavone-7-O-D-glucoside (baicaleine-7-O-D-glucoside), 5-hydroxy-4',6,7-trimethoxy-flavone (or salvigenine), 5-hydroxy-6-methoxy-flavone-7-O-D-glucuronide (or oroxyline A-7-O-D-glucuronide), 5,6-dihydroxy-flavone-7-O-D-glucuronide (or baicaline), 5-hydroxy-6-methoxy-flavone-7-O-D-glucuronic methyl ester acid (or oroxindine methyl ester), 5,7-dihydroxy-flavone (or chrysine).

2. Composition according to Claim 1, characterized in that said active substance is selected from the group constituted by wogonine, 2'-hydroxy-wogonine, baicaleine, neobaicaleine, oroxindine and baicaline.

3. Composition according to Claim 1, characterized in that the said extract is obtained by an extraction by solvent, preferably selected from the group consisting of a polar solvent, particularly an alcoholic or dilute alcoholic solution, or an ethereal solution, of a nonpolar organic solvent, such as n-hexane, benzene, or advantageously a combination of the two.

4. Composition according to one of Claims 1 to 3, characterized in that the said extract alone or the active substance isolated from such an extract or obtained by chemical synthesis of such an extract, is used alone or mixed with other compatible active substances.

5. Composition according to one of Claims 1 to 4, characterized in that the said extract alone or said active substance isolated from such an extract or obtained by chemical synthesis is introduced into the lipidic phase of the hydrated lipidic lamellar phases or of the liposomes.

6. Composition according to one of Claims 1 to 4, characterized in that the said extract or the active substance isolated from such an extract or obtained by chemical synthesis mentioned above is introduced into the aqueous phase of the hydrated lipidic lamellar phases or of the liposomes.

7. Composition according to any one of Claims 1 to 6, characterized in that the Scutellaria is selected from the group consisting of Scutellaria Baicalensis, Scutellaria Viscidula or Scutellaria Galericulata.

8. Composition according to one of Claims 1 to 7, characterized in that the extract of Scutellaria is an extract of roots of Scutellaria Baicalensis Georgi, also called extract of Ogon.

9. Cosmetic or pharmaceutical, particularly dermatological composition, with anti-inflammatory, anti-allergic or anti-ageing activity, characterized in that it comprises a composition based on hydrated lipidic lamellar phases or liposomes, containing at least partly an extract of Scutellaria, or at least one active substance isolated from such an extract or obtained by chemical synthesis, selected from: 2′,5,7-trihydroxy-8-methoxy flavone (or 2′-hydroxy-wogonine), 2′5-dihydroxy-(6,6′,7,8-tetramethoxy-flavone (or skullcap flavone II or neobaicaleine), 2′,5,5′,7-tetrahydroxy-6′,8-dimethoxy-flavone, 5-hydroxy-8-methoxy-flavone-7-O-D-glucuronide (or wogonine-7-O-D-glucuronide or oroxindine), 5-hydroxy-7,8-dimethoxy-flavone (or 7-O-methyl-wogonine), 5,7-dihydroxy-6-methoxy-flavone (or oroxyline A or 6-O-methyl-baicaleine), 4′,5,7-trihydroxy-8-methoxy-flavone (or 4′-hydroxy-wogonine), 2′,5,6′-trihydroxy-7,8-dimethoxy-flavone, 5,7,8-trihydroxy-flavone (or norwogonine), 5,6,7-trihydroxy-flavone (or baicaleine), 5,8-dihydroxy-6,7-dimethoxy-flavone, 2′,3,5,6′,7-pentahydroxy-flavone, 4′,5,6-trihydroxy-flavone-7-O-D-glucuronide (or 4′-hydroxy-baicaline), 5,6-dihydroxy-flavone-7-O-D-glucuronic methyl ester acid (or methyl ester baicaline), 2′,5,7-trihydroxy-flavone (2′-hydroxy chrysine), 5,7-dihydroxy-8-methoxy-flavone (or wogonine), 2′,5,7-trihydroxy-6′,8-dimethoxy-flavone (or 2′-hydroxy-6′- methoxy-wogonine), 4′,5,6,7-tetrahydroxy-flavone (or 4′-hydroxy-baicaleine), 5,6-dihydroxy-flavone-7-O-D-glucoside (baicaleine-7-O-D-glucoside), 5-hydroxy-4′,6,7-trimethoxy-flavone (or salvigenine), 5-hydroxy-6-methoxy-flavone-7-O-D-glucuronide (or oroxyline A-7-O-D-glucuronide), 5,6-dihydroxy-flavone-7-O-D-glucuronide (or baicaline), 5-hydroxy-6-methoxy-flavone-7-O-D-glucuronic methyl ester acid (or oroxindine methyl ester), 5,7-dihydroxy-flavone (or chrysine).

10. Cosmetic or pharmaceutical composition according to Claim 9, characterized in that said active substance is selected from the group constituted by wogonine, 2′-hydroxy-wogonine, baicaleine, neobaicaleine, oroxindine and baicaline.

11. Cosmetic or pharmaceutical composition according to Claim 9 or 10, characterized in that the said extract is obtained by an extraction by solvent, preferably selected from the group consisting of a polar solvent, particularly an alcoholic or dilute alcoholic solution, or an ethereal solution, of a nonpolar organic solvent, such as n-hexane, benzene, or advantageously a combination of the two.

12. Cosmetic or pharmaceutical composition according to one of Claims 9 to 11, characterized in that the said extract alone or said active substance isolated from such an extract or obtained by chemical synthesis, is introduced alone or mixed with other compatible active substances in the lipidic phase of the hydrated lipidic lamellar phases or of the liposomes.

13. Cosmetic or pharmaceutical composition according to one of Claims 9 to !2, characterized in that the said extract or the active substance isolated from such an extract or obtained by chemical synthesis is introduced alone or mixed with other compatible active substances in the aqueous phase of the hydrated lipidic lamellar phases or of the liposomes.

14. Cosmetic or pharmaceutical composition according to Claims 9 to 13, characterized in that the Scutellaria is selected from the group consisting of Scutellaria Baicalensis, Scutellaria Viscidula or Scutellaria Galericulata.

15. Cosmetic or pharmaceutical composition according to Claims 9 to 14, characterized in that the extract of Scutellaria is an extract of roots of Scutellaria Baicalensis Georgi, also called extract of Ogon.

16. Pharmaceutical, particularly dermatological, or cosmetic composition according to one of Claims 9 to 15, characterized in that the proportion by weight of extract of Scutellaria, expressed in dry extract or of the active substance isolated from such an extract or obtained by chemical synthesis mentioned above, is included between 0.0001 and 2%, preferably between 0.001 and 0.4% by weight with respect to the total weight of the composition.

## Claims for the following Contrating State: ES

1. Process for preparing composition based on hydrated lipidic lamellar phases or liposomes, characterized in that it consists in incorporating at least partly an extract of Scutellaria, or at least one active substance isolated from such an extract or obtained by chemical synthesis, selected from: 2',5,7-trihydroxy-8-methoxy flavone (or 2'-hydroxy-wogonine), 2'5-dihydroxy-(6,6',7,8-tetramethoxy-flavone (or skullcap flavone II or neobaicaleine), 2',5,5',7-tetrahydroxy-6',8-dimethoxy-flavone, 5-hydroxy-8-methoxy-flavone-7-O-D-glucuronide (or wogonine-7-O-D-glucuronide or oroxindine), 5-hydroxy-7,8-dimethoxy-flavone (or 7-O-methyl-wogonine), 5,7-dihydroxy-6-methoxy-flavone (or oroxyline A or 6-O-methyl-baicaleine), 4',5,7-trihydroxy-8-methoxy-flavone (or 4'-hydroxy-wogonine), 2',5,6'-trihydroxy-7,8dimethoxy-flavone, 5,7,8-trihydroxy-flavone (or norwogonine), 5,6, 7-trihydroxy-flavone (or baicaleine), 5,8-dihydroxy-6,7-dimethoxy-flavone, 2',3,5,6',7-pentahydroxy-flavone, 4',5,6-trihydroxy-flavone-7-O-D-glucuronide (or 4'-hydroxy-baicaline), 5,6-dihydroxy-flavone-7-O-D-glucuronic methyl ester acid (or methyl ester baicaline), 2',5,7-trihydroxy-flavone (2'-hydroxy chrysine), 5,7-dihydroxy-8-methoxy-flavone (or wogonine), 2',5,7-trihydroxy-6',8-dimethoxy-flavone (or 2'-hydroxy-6'-methoxy-wogonine), 4',5,6,7-tetrahydroxy-flavone (or 4'-hydroxy-baicaleine), 5,6-dihydroxy-flavone-7-O-D-glucoside (baicaleine-7-O-D-glucoside), 5-hydroxy-4',6,7-trimethoxy-flavone (or salvigenine), 5-hydroxy-6-methoxy-flavone-7-O-D-glucuronide (or oroxyline A-7-O-D-glucuronide), 5,6-dihydroxy-flavone-7-O-D-glucuronide (or baicaline), 5-hydroxy-6-methoxy-flavone-7-O-D-glucuronic methyl ester acid (or oroxindine methyl ester), 5,7-dihydroxy-flavone (or chrysine), in said hydrated lipidic lamellar phases or said liposomes.

2. Process according to Claim 1, characterized in that said active substance is selected from the group constituted by wogonine, 2'-hydroxy-wogonine, baicaleine, neobaicaleine, oroxindine and baicaline.

3. Process according to Claim 1, characterized in that the said extract is obtained by an extraction by solvent, preferably selected from the group consisting of a polar solvent, particularly an alcoholic or dilute alcoholic solution, or an ethereal solution, of a nonpolar organic solvent, such as n-hexane, benzene, or advantageously a combination of the two.

4. Process according to one of Claims 1 to 3, characterized in that the said extract alone or the active substance isolated from such an extract or obtained by chemical synthesis of such an extract, is used alone or mixed with other compatible active substances.

5. Process according to one of Claims 1 to 4, characterized in that the said extract alone or said active substance isolated from such an extract or obtained by chemical synthesis is introduced into the lipidic phase of the hydrated lipidic lamellar phases or of the liposomes.

6. Process according to one of Claims 1 to 4, characterized in that the said extract or the active substance isolated from such an extract or obtained by chemical synthesis mentioned above is introduced into the aqueous phase of the hydrated lipidic lamellar phases or of the liposomes.

7. Process according to any one of Claims 1 to 6, characterized in that the Scutellaria is selected from the group consisting of Scutellaria Baicalensis, Scutellaria Viscidula or Scutellaria Galericulata.

8. Process according to one of Claims 1 to 7, characterized in that the extract of Scutellaria is an extract of roots of Scutellaria Baicalensis Georgi, also called extract of Ogon.

9. Process for preparing a cosmetic or pharmaceutical, particularly dermatological composition, with anti-inflammatory, anti-allergic or anti-ageing activity, characterized in that it consists in incorporating a composition based on hydrated lipidic lamellar phases or liposomes, containing at least partly an extract of Scutellaria, or at least one active substance isolated from such an extract or obtained by chemical synthesis, selected from: 2',5,7-trihydroxy-8-methoxy flavone (or 2'-hydroxy-wogonine), 2'5-dihydroxy-(6,6',7,8-tetramethoxy-flavone (or skullcap flavone II or neobaicaleine), 2',5,5',7-tetrahydroxy-6',8-dimethoxy-flavone, 5-hydroxy-8-methoxy-flavone-7-O-D-glucuronide (or wogonine-7-O-D-glucuronide or oroxindine), 5-hydroxy-7,8-dimethoxy-flavone (or 7-O-methyl-wogonine), 5,7-dihydroxy-6-methoxy-flavone (or oroxyline A or 6-O-methyl-baicaleine), 4',5,7-trihydroxy-8-methoxy-flavone (or 4'-hydroxy-wogonine), 2',5,6'-trihydroxy-7,8-dimethoxy-flavone, 5,7,8-trihydroxy-flavone (or norwogonine), 5,6, 7-trihydroxy-flavone (or baicaleine), 5,8-dihydroxy-6,7-dimethoxy-flavone, 2',3,5,6',7-pentahydroxy-flavone, 4',5,6-trihydroxy-flavone-7-O-D-glucuronide (or 4'-hydroxy-baicaline), 5,6-dihydroxy-flavone-7-O-D-glucuronic methyl ester acid (or methyl ester baicaline), 2',5,7-trihydroxy-flavone (2'-hydroxy chrysine), 5,7-dihydroxy-8-methoxy-flavone (or wogonine), 2',5,7-trihydroxy-6',8-dimethoxy-flavone (or 2'-hydroxy-6'-methoxy-wogonine), 4',5,6,7-tetrahydroxy-flavone (or 4'-hydroxy-baicaleine), 5,6-dihydroxy-flavone-7-O-D-glucoside (baicaleine-7-O-D-glucoside), 5-hydroxy-4',6,7-trimethoxy-flavone (or salvigenine), 5-hydroxy-6-methoxy-flavone-7-O-D-glucuronide (or oroxyline A-7-O-D-glucuronide), 5,6-dihydroxy-flavone-7-O-D-glucuronide (or baicaline), 5-hydroxy-6-methoxy-flavone-7-O-D-glucuronic methyl ester acid (or oroxindine methyl ester), 5,7-dihydroxy-flavone (or chrysine) prepared beforehand, in a cosmetically or pharmaceutical acceptable excipient or base.

10. Process according to Claim 9, characterized in that said active substance is selected from the group constituted by wogonine, 2'-hydroxy-wogonine, baicaleine, neobaicaleine, oroxindine and baicaline

11. Process according to Claim 9 or 10, characterized in that the said extract is obtained by an extraction by solvent, preferably selected from the group consisting of a polar solvent, particularly an alcoholic or dilute alcoholic solution, or an ethereal solution, of a nonpolar organic solvent, such as n-hexane, benzene, or advantageously a combination of the two.

12. Process according to one of Claims 9 to 11, characterized in that the said extract alone or said active substance isolated from such an extract or obtained by chemical synthesis, is introduced alone or mixed with other compatible active substances in the lipidic phase of the hydrated lipidic lamellar phases or of the liposomes.

13. Process according to one of Claims 9 to 12, characterized in that the said extract or the active substance isolated from such an extract or obtained by chemical synthesis is introduced alone or mixed with other compatible active substances in the aqueous phase of the hydrated lipidic lamellar phases or of the liposomes.

14. Process according to Claims 9 to 13, characterized in that the Scutellaria is selected from the group consisting of Scartellaria Baicalensis, Scutellaria Viscidula or Scutelleria Galericulata.

15. Process according to any one of claims 9 to 14, characterized in that the extract of Scutellaria is an extract of roots of Scutellaria Baicalensis Georgi, also called extract of Ogon.

16. Process according to one of Claims 9 to 15, characterized in that the proportion by weight of extract of Scutellaria, expressed in dry extract or of the active substance isolated from such an extract or obtained by chemical synthesis mentioned above, is included between 0.0001 and 2%, preferably between 0.001 and 0.4% by weight with respect to the total weight of the composition.

**Claims for the following Contracting State: GR**

1. Composition based on hydrated lipidic lamellar phases or liposomes, characterized in that said hydrated lipidic lamellar phases or said liposomes contain at least partly an extract of Scutellaria, or at least one active substance isolated from such an extract or obtained by chemical synthesis, selected from: 2',5,7-trihydroxy-8-methoxy flavone (or 2'-hydroxy-wogonine), 2'5-dihydroxy-(6,6',7,8-tetramethoxy-flavone (or skullcap flavone II or neobaicaleine), 2',5,5',7-tetrahydroxy-6',8-dimethoxy-flavone, 5-hydroxy-8-methoxy-flavone-7-O-D-glucuronide (or wogonine-7-O-D-glucuronide or oroxindine), 5-hydroxy-7,8-dimethoxy-flavone (or 7-O-methyl-wogonine), 5,7-dihydroxy-6-methoxy-flavone (or oroxyline A or 6-O-methyl-baicaleine), 4',5,7-trihydroxy-8-methoxy-flavone (or 4'-hydroxy-wogonine), 2',5,6'-trihydroxy-7,8-dimethoxy-flavone, 5,7,8-trihydroxy-flavone (or norwogonine), 5,6, 7-trihydroxy-flavone (or baicaleine), 5,8-dihydroxy-6,7-dimethoxy-flavone, 2',3,5,6',7-pentahydroxy-flavone, 4',5,6-trihydroxy-flavone-7-O-D-glucuronide (or 4'-hydroxy-baicaline), 5,6-dihydroxy-flavone-7-O-D-glucuronic methyl ester acid (or methyl ester baicaline), 2',5,7-trihydroxy-flavone (2'-hydroxy chrysine), 5,7-dihydroxy-8-methoxy-flavone (or wogonine), 2',5,7-trihydroxy-6',8-dimethoxy-flavone (or 2'-hydroxy-6'-methoxy-wogonine), 4',5,6,7-tetrahydroxy-flavone (or 4'-hydroxy-baicaleine), 5,6-dihydroxy-flavone-7-O-D-glucoside (baicaleine-7-O-D-glucoside), 5-hydroxy-4',6,7-trimethoxy-flavone (or salvigenine), 5-hydroxy-6-methoxy-flavone-7-O-D-glucuronide (or oroxyline A-7-O-D-glucuronide), 5,6-dihydroxy-flavone-7-O-D-glucuronide (or baicaline), 5-hydroxy-6-methoxy-flavone-7-O-D-glucuronic methyl ester acid (or oroxindine methyl ester), 5,7-dihydroxy-flavone (or chrysine).

2. Composition according to Claim 1, characterized in that said active substance is selected from the group constituted by wogonine, 2'-hydroxy-wogonine, baicaleine, neobaicaleine, oroxindine and baicaline.

3. Composition according to Claim 1, characterized in that the said extract is obtained by an extraction by solvent, preferably selected from the group consisting of a polar solvent, particularly an alcoholic or dilute alcoholic solution, or an ethereal solution, of a nonpolar organic solvent, such as n-hexane, benzene, or advantageously a combination of the two.

4. Composition according to one of Claims 1 to 3, characterized in that the said extract alone or the active substance isolated from such an extract or obtained by chemical synthesis of such an extract, is used alone or mixed with other compatible active substances.

5. Composition according to one of Claims 1 to 4, characterized in that the said extract alone or said active substance isolated from such an extract or obtained by chemical synthesis is introduced into the lipidic phase of the hydrated lipidic lamellar phases or of the liposomes.

6. Composition according to one of Claims 1 to 4, characterized in that the said extract or the active substance isolated from such an extract or obtained by chemical synthesis mentioned above is introduced into the aqueous phase of the hydrated lipidic lamellar phases or of the liposomes.

7. Composition according to any one of Claims 1 to 6, characterized in that the Scutellaria is selected from the group consisting of Scutellaria Baicalensis, Scutellaria Viscidula or Scutellaria Galericulata.

8. Composition according to one of Claims 1 to 7, characterized in that the extract of Scutellaria is an extract of roots of Scutellaria Baicalensis Georgi, also called extract of Ogon.

9. Cosmetic composition, characterized in that it comprises a composition based on hydrated lipidic lamellar phases or liposomes, containing at least partly an extract of Scutellaria, or at least one active substance isolated from such an extract or obtained by chemical synthesis, selected from: 2′,5,7-trihydroxy-8-methoxy flavone (or 2′-hydroxy-wogonine), 2′5-dihydroxy-(6,6′,7,8-tetramethoxy-flavone (or skullcap flavone II or neobaicaleine), 2′,5,5′,7-tetrahydroxy-6′,8-dimethoxy-flavone, 5-hydroxy-8-methoxy-flavone-7-O-D-glucuronide (or wogonine-7-O-D-glucuronide or oroxindine), 5-hydroxy-7,8-dimethoxy-flavone (or 7-O-methyl-wogonine), 5,7-dihydroxy-6-methoxy-flavone (or oroxyline A or 6-O-methyl-baicaleine), 4′,5,7-trihydroxy-8-methoxy-flavone (or 4′-hydroxy-wogonine), 2′,5,6′-trihydroxy-7,8-dimethoxy-flavone, 5,7,8-trihydroxy-flavone (or norwogonine), 5,6, 7-trihydroxy-flavone (or baicaleine), 5,8-dihydroxy-6,7-dimethoxy-flavone, 2′,3,5,6′,7-pentahydroxy-flavone, 4′,5,6-trihydroxy-flavone-7-O-D-glucuronide (or 4′-hydroxy-baicaline), 5,6-dihydroxy-flavone-7-O-D-glucuronic methyl ester acid (or methyl ester baicaline), 2′,5,7-trihydroxy-flavone (2′-hydroxy chrysine), 5,7-dihydroxy-8-methoxy-flavone (or wogonine), 2′,5,7-trihydroxy-6′,8-dimethoxy-flavone (or 2′-hydroxy-6′-methoxy-wogonine), 4′,5,6,7-tetrahydroxy-flavone (or 4′-hydroxy-baicaleine), 5,6-dihydroxy-flavone-7-O-D-glucoside (baicaleine-7-O-D-glucoside), 5-hydroxy-4′,6,7-trimethoxy-flavone (or salvigenine), 5-hydroxy-6-methoxy-flavone-7-O-D-glucuronide (or oroxyline A-7-O-D-glucuronide), 5,6-dihydroxy-flavone-7-O-D-glucuronide (or baicaline), 5-hydroxy-6-methoxy-flavone-7-O-D-glucuronic methyl ester acid (or oroxindine methyl ester), 5,7-dihydroxy-flavone (or chrysine).

10. Cosmetic composition according to Claim 9, characterized in that said active substance is selected from the group constituted by wogonine, 2′-hydroxy-wogonine, baicaleine, neobaicaleine, oroxindine and baicaline.

11. Cosmetic composition according to Claim 9 or 10, characterized in that the said extract is obtained by an extraction by solvent, preferably selected from the group consisting of a polar solvent, particularly an alcoholic or dilute alcoholic solution, or an ethereal solution, of a nonpolar organic solvent, such as n-hexane, benzene, or advantageously a combination of the two.

12. Cosmetic composition according to one of Claims 9 to 11, characterized in that the said extract alone or said active substance isolated from such an extract or obtained by chemical synthesis, is introduced alone or mixed with other compatible active substances in the lipidic phase of the hydrated lipidic lamellar phases or of the liposomes.

13. Cosmetic composition according to one of Claims 9 to 12, characterized in that the said extract or the active substance isolated from such an extract or obtained by chemical synthesis is introduced alone or mixed with other compatible active substances in the aqueous phase of the hydrated lipidic lamellar phases or of the liposomes.

14. Cosmetic composition according to Claims 9 to 13, characterized in that the Scutellaria is selected from the group consisting of Scutellaria Baicalensis, Scutellaria Viscidula or Scutellaria Galericulata.

15. Cosmetic composition according to Claims 9 to 14, characterized in that the extract of Scutellaria is an extract of roots of Scutellaria Baicalensis Georgi, also called extract of Ogon.

16. Cosmetic composition according to one of Claims 9 to 15, characterized in that the proportion by weight of extract of Scutellaria, expressed in dry extract or of the active substance isolated from such an extract or obtained by chemical synthesis mentioned above, is included between 0.0001 and 2%, preferably between 0.001 and 0.4% by weight with respect to the total weight of the composition.

17. Process for preparing a cosmetic or pharmaceutical composition, with anti-inflammatory, anti-allergic or anti-ageing activity, characterized in that it consists in incorporating a composition based on hydrated lipidic lamellar phases or liposomes, containing at least partly an extract of Scutellaria, or at least one active substance isolated from such an extract or obtained by chemical synthesis, selected from: 2′,5,7-trihydroxy-8-methoxy flavone (or 2′-hydroxy-wogonine), 2′5-dihydroxy-(6,6′,7,8-tetramethoxy-flavone (or skullcap flavone II or neobaicaleine), 2′,5,5′,7-tetrahydroxy-6′,8-dimethoxy-flavone, 5-hydroxy-8-methoxy-flavone-7-O-D-glucuronide (or wogonine-7-O-D-glucuronide or oroxindine), 5-hydroxy-7,8-dimethoxy-flavone (or 7-O-methyl-wogonine), 5,7-dihydroxy-6-methoxy-flavone (or oroxyline A or 6-O-methyl-baicaleine), 4′,5,7-trihydroxy-8-methoxy-flavone (or 4′-hydroxy-wogonine), 2′,5,6′-trihydroxy-7,8-dimethoxy-flavone, 5,7,8-trihydroxy-flavone (or norwogonine), 5,6,7-trihydroxy-flavone (or baicaleine), 5,8-dihydroxy-6,7-dimethoxy-flavone, 2′,3,5,6′,7-pentahydroxy-flavone, 4′,5,6-trihydroxy-flavone-7-O-D-glucuronide (or 4′-hydroxy-baicaline), 5,6-dihydroxy-flavone-7-O-D-glucuronic methyl ester acid (or methyl ester baicaline), 2′,5,7-trihydroxy-flavone (2′-hydroxy chrysine), 5,7-dihydroxy-8-methoxy-flavone (or wogonine), 2′,5,7-trihydroxy-6′,8-dimethoxy-flavone (or 2′-hydroxy-6′-methoxywogonine), 4′,5,6,7-tetrahydroxy-flavone (or 4′-hydroxy-baicaleine), 5,6-dihydroxy-flavone-7-O-D-glucoside (baicaleine-7-O-D-glucoside), 5-hydroxy-4′,6,7-trimethoxy-flavone (or salvigenide), 5-hydroxy-6-methoxy-flavone-7-O-D-glucuronide (or oroxyline A-7-O-D-glucuronide), 5,6-dihydroxy-flavone-7-O-D-glucuronide (or baicaline), 5-hydroxy-6-methoxy-flavone-7-O-D-glucuronic methyl ester acid (or oroxindine methyl ester), 5,7-dihydroxy-flavone (or chrysine), prepared beforehend, in a cosmetically or pharmaceutically acceptable excipient or base.

ACTIVITE ANTIPHLOGISTIQUE